# EUROPEAN PATENT SPECIFICATION

(11) **EP 2 528 002 B1**
(45) Date of publication and mention of the grant of the patent: **04.11.2015**
(21) Application number: 12158671.3
(22) Date of filing: 15.04.2008
(51) Int. Cl.: G06F 19/28, C12P 21/00

(54) **Defined glycoprotein products and related methods**
Definierte Glycoproteinprodukte und entsprechende Verfahren
Produits de glycoprotéine définie et procédés associés

(30) Priority: 16.04.2007 US 912102 P
(43) Date of publication of application: 28.11.2012
(62) Divisional of application: 08754908.5
(73) Proprietor: Momenta Pharmaceuticals, Inc., Cambridge, MA 02142 (US)
(72) Inventor: Venkataraman, Ganesh, Bedford, MA 01730 (US); Collins, Brian, Edward, Arlington, MA 02474 (US); Bosques, Carlos, J., Arlington, MA 02476 (US); Thiruneelakantapillai, Lakshmanan, Boston, MA 02125 (US); Bulik, Dorota, A., Malden, MA 02148 (US); Parsons, Ian Christopher, Belmont, MA 02478 (US); Shriver, Zachary, Cambridge, MA 02139 (US); Chillakuru, Rajeev, Cambridge, MA 02142 (US)
(74) Representative: Simpson, Tobias Rutger

(56) References cited:
- WO-A2-00/65070
- GAWLITZEK MARTIN ET AL: "AMMONIUM ALTERS N-GLYCAN STRUCTURES OF RECOMBINANT TNFR-IGG: DEGRADATIVE VERSUS BIOSYNTHETIC MECHANISMS", BIOTECHNOLOGY AND BIOENGINEERING, WILEY & SONS, HOBOKEN, NJ, US, vol. 68, no. 6, 20 June 2000 (2000-06-20), pages 637-646, XP008079772, ISSN: 0006-3592
- CHEN P ET AL: "Effects of elevated ammonium on glycosylation gene expression in CHO cells", METABOLIC ENGINEERING, ACADEMIC PRESS, US, vol. 8, no. 2, 1 March 2006 (2006-03-01), pages 123-132, XP024946928, ISSN: 1096-7176, DOI: 10.1016/J.YMBEN.2005.10.002 [retrieved on 2006-03-01]
- CROWELL CHRISTOPHER K ET AL: "Amino acid and manganese supplementation modulates the glycosylation state of erythropoietin in a CHO culture system", BIOTECHNOLOGY AND BIOENGINEERING, WILEY & SONS, HOBOKEN, NJ, US, vol. 96, no. 3, 1 February 2007 (2007-02-01), pages 538-549, XP002454615, ISSN: 0006-3592, DOI: 10.1002/BIT.21141
- COOPER CATHERINE A ET AL: "GlycoSuiteDB: A curated relational database of glycoprotein glycan structures and their biological sources. 2003 update.", NUCLEIC ACIDS RESEARCH, vol. 31, no. 1, 1 January 2003 (2003-01-01), pages 511-513, XP002486792, ISSN: 0305-1048
- HOSOI S ET AL: "MODULATION OF OLIGOSACCHARIDE STRUCTURE OF A PRO-UROKINASE DERIVATIVE (PRO-UKDELTAGS1) BY CHANGING CULTURE CONDITIONS OF A LYMPHOBLASTOID CELL LINE NAMALWA KJM-1 ADAPTED TO SERUM-FREE MEDIUM", CYTOTECHNOLOGY, KLUWER ACADEMIC PUBLISHERS, DORDRECHT, NL, vol. 19, no. 2, 1 January 1996 (1996-01-01), pages 125-135, XP000971674, ISSN: 0920-9069, DOI: 10.1007/BF00749767
- GAWLITZEK M ET AL: "Characterization of changes in the glycosylation pattern of recombinant proteins from BHK-21 cells due to different culture conditions", JOURNAL OF BIOTECHNOLOGY, ELSEVIER SCIENCE PUBLISHERS, AMSTERDAM, NL, vol. 42, no. 2, 29 September 1995 (1995-09-29), pages 117-131, XP004036908, ISSN: 0168-1656, DOI: 10.1016/0168-1656(95)00065-X
- JONG HYUN NAM ET AL: "The effects of culture conditions on the glycosylation of secreted human placental alkaline phosphatase produced in Chinese hamster ovary cells", BIOTECHNOLOGY AND BIOENGINEERING, vol. 100, no. 6, 4 March 2008 (2008-03-04) , pages 1178-1192, XP055053835, ISSN: 0006-3592, DOI: 10.1002/bit.21853
- HENDRICK V ET AL: "Increased productivity of recombinant tissular plasminogen activator (t-PA) by butyrate and shift of temperature: a cell cycle phases analysis", CYTOTECHNOLOGY, KLUWER ACADEMIC PUBLISHERS, DO, vol. 36, no. 1-3, 1 July 2001 (2001-07-01) , pages 71-83, XP019236697, ISSN: 1573-0778, DOI: 10.1023/A:1014088919546
- ROBINSON D K ET AL: "Characterization of a recombinant antibody produced in the course of a high yield fed-batch process", BIOTECHNOLOGY AND BIOENGINEERING, WILEY & SONS, HOBOKEN, NJ, US, vol. 44, no. 6, 5 September 1994 (1994-09-05), pages 727-735, XP002559794, ISSN: 0006-3592, DOI: 10.1002/BIT.260440609 [retrieved on 2004-02-19]
- C.E. JOOSTEN ET AL: "Effect of Culture Conditions on the Degree of Sialylation of a Recombinant Glycoprotein Expressed in Insect Cells", BIOTECHNOLOGY PROGRESS, vol. 19, no. 3, 6 June 2003 (2003-06-06), pages 739-749, XP055053952, ISSN: 8756-7938, DOI: 10.1021/bp0201049
- YANG M ET AL: "Effects of Ammonia and Glucoseamine on the Heterogeneity of Erythropoietin Glycoforms", BIOTECHNOLOGY PROGRESS, AMERICAN INSTITUTE OF CHEMICAL ENGINEERS, US, vol. 18, no. 1, 1 January 2002 (2002-01-01), pages 129-138, XP002997170, ISSN: 8756-7938, DOI: 10.1021/BP0101334
- RODRIGUEZ J ET AL: "Enhanced production of monomeric interferon-[beta] by CHO cells through the control of culture conditions", BIOTECHNOLOGY PROGRESS, AMERICAN INSTITUTE OF CHEMICAL ENGINEERS, US, vol. 21, no. 1, 1 January 2005 (2005-01-01), pages 22-30, XP002572995, ISSN: 8756-7938, DOI: 10.1021/BP049807B [retrieved on 2004-12-29]
- LIFELY M R (REPRINT) ET AL: "Glycosylation and biological-activity of CAMPATH-1H expressed in different cell-lines and grown under different culture conditions", GLYCOBIOLOGY, OXFORD UNIVERSITY PRESS, US, vol. 5, no. 8, 1 December 1995 (1995-12-01), pages 813-822, XP002096118, ISSN: 0959-6658, DOI: 10.1093/GLYCOB/5.8.813
- COX KEVIN M ET AL: "GLYCAN OPTIMIZATION OF A HUMAN MONOCLONAL ANTIBODY IN THE AQUATIC PLANT LEMNA MINOR", NATURE BIOTECHNOLOGY, NATURE PUBLISHING GROUP, NEW YORK, NY, US, vol. 24, no. 12, 1 December 2006 (2006-12-01), pages 1591-1597, XP009084966, ISSN: 1087-0156, DOI: 10.1038/NBT1260
- Ingrid M. Van Den Nieuwenhof ET AL: "Recombinant glycodelin carrying the same type of glycan structures as contraceptive glycodelin-A can be produced in human kidney 293 cellsbut not in Chinese hamster ovary cells", European Journal of Biochemistry, vol. 267, no. 15, 25 August 2000 (2000-08-25), pages 4753-4762, XP055087225, ISSN: 0014-2956, DOI: 10.1046/j.1432-1327.2000.01528.x
- M Gawlitzek ET AL: "Ammonium ion and glucosamine dependent increases of oligosaccharide complexity in recombinant glycoproteins secreted from cultivated BHK-21 cells", Biotechnology and bioengineering, 5 March 1998 (1998-03-05), pages 518-528, XP055087229, UNITED STATES DOI: 10.1002/(SICI)1097-0290(19980305)57:5<518: :AID-BIT3>3.0.CO;2-J Retrieved from the Internet: URL:http://www.ncbi.nlm.nih.gov/pubmed/100 99230

## Description

The present disclosure relates to glycoprotein products and related methods, e.g., methods of making reference glycoprotein products and methods of designing processes to make glycoprotein products having defined physical and functional properties.

### BACKGROUND

Many drugs in use today are "small molecule drugs." These drugs exist as simple chemical structures that are synthetically derived. The active ingredient generally exists as a homogenous product. These small molecule drugs and preparations thereof can be chemically characterized and are generally readily manufactured through comparatively simple chemical synthesis.

A typical glycoprotein product differs substantially in terms of complexity from a typical small molecule drug. The sugar structures attached to the amino acid backbone of a glycoprotein can vary structurally in many ways including, sequence, branching, sugar content, and heterogeneity. Thus, glycoprotein products can be complex heterogeneous mixtures of many structurally diverse molecules which themselves have complex glycan structures. Glycosylation adds not only to the molecule's structural complexity but affects or conditions many of a glycoprotein's biological and clinical attributes.

To date, the creation of glycoprotein drugs having defined properties, whether an attempt to produce a generic version of an existing drug or to produce a second generation or other glycoprotein having improved or desirable properties has been scientifically challenging due to the difficulty in understanding and synthesizing these complex chemical structures and mixtures that contain them.

The situation with regard to the production of generic products is indicative of the problems faced in making glycoprotein drugs having defined properties. While abbreviated regulatory procedures have been implemented for generic versions of drug products, many in the biotechnology and pharmaceutical industry have taken the view that the complexity of biological products makes them unsuitable for similar approaches.

Van Den Nieuwenhof et al., Eur. J. Biochem., 267, 4753-4762, 2000 disclose that recombinant glycodelin carrying the same type of glycan structures as contraceptive glycodelin-A can be produced in human kidney 293 cells but not in Chinese hamster ovary cells.

Gawlitzek et al., Biotechnology and Bioengineering, 57(5), 518-528, 1998 disclose ammonium ion and glucosamine dependent increases of oligosaccharide complexity in recombinant glycoproteins secreted from cultivated baby hamster kidney 21 cells.

### SUMMARY

### Methods of Making Glycoproteins

Methods disclosed herein allow for the production of glycoproteins having defined glycan structures and/or defined glycan mediated functional properties. Some methods rely on the use of databases which include correlations between production parameters and desired glycan properties. The database can provide production parameters for incorporation into a production protocol. The methods allows for the production of designed glycoproteins or in general glycoproteins having defined glycan properties.

According to the present invention, there is provided a method of producing a protein with a modulated amount of a glycan characteristic by modulating a production parameter, said method comprising:
selecting a reference level of said glycan characteristic;
selecting a value for a production parameter to provide a modulated level of said glycan characteristic; and
applying the selected value of said production parameter in a process for making a protein with a modulated amount of said glycan characteristic, wherein said process for making the protein comprises culturing a CHO cell in a cell culture medium;
wherein:
   said glycan characteristic is fucosylation and said modulated amount of said glycan characteristic is a decreased level of fucosylation; and
   said production parameter is the glucosamine content of the cell culture medium and said value for said production parameter is a glucosamine concentration of 0.001 to 40 mM.

More generally, the present disclosure features, a method for making a glycoprotein product including the steps of:
i) providing a database that correlates, defines, identifies, relates, or provides each of a plurality of glycan properties as a correlative function of one or more production parameters or combinations of production parameters;
ii) identifying a target glycan property, e.g., a glycan property of a primary glycoprotein product;
iii) selecting from the database one or more production parameters or combinations of production parameters that correlate with the target glycan property; and
iv) applying the selected production parameter or combinations of production parameters in a process for making the glycoprotein product,
thereby making a glycoprotein product.

As discussed in detail elsewhere herein, methods, databases, and systems disclosed herein can include or use various types of correlations between production parameters and the glycan properties they condition. These are referred to as correlative functions. The production of glycoproteins is a complex process and correlations provided in the databases can reflect this. Exemplary correlative functions include non-linear correlative functions. A nonlinear correlation can reflect a relationship between production parameters and glycan properties wherein the effect of two (or more) production parameters acting together on a glycan property is not the same as the combination of a first production parameter (acting alone) on the glycan property together with the effect of a second production parameter (acting alone) on the glycan property. This can be expressed as:
X1→ Y1; X2→ Y2; X1+X2 ≠ →Y1+Y2, e.g., X1+X2→Y3, in the notation used herein.

Other types of correlative functions useful in the methods, databases and systems described herein include constrained, pleiotropic and tunable correlative functions. Briefly, constrained correlative functions reflect the complexity of glycoprotein synthesis and can represent relationships characterized by incompatible or undesirable combinations or production parameters or glycan properties. E.g., a combination of production parameters may be constrained because it results in an undesirable glycan property. Pleiotropic correlative functions can reflect the varied effect of one or more production parameter on different glycan characteristics. A tunable function is one that can allow for a plurality of inputs, e.g., inputs of differing magnitudes, and a plurality of outputs, e.g., of differing magnitude. It can allow the adjustment of a glycan property by the adjustment of a production parameter. These and other correlative functions are discussed in more detailed below.

Accordingly, in an instance of the disclosure the database includes ten or more, e.g., 20, 25, 50, 100, 150, 200, 300, 350, 400, 500, 600, 700, 800, 900 or more, tunable, nonlinear, pleiotropic, or constrained correlations. In an instance a selected production parameter is associated with a tunable, nonlinear, pleiotropic, or constrained correlation.

In an instance a first production parameter X1 is selected by a correlative function between production parameter X1 and a glycan property Y1 and a glycan property Y2 and a second production parameter X2 is selected to modify the effect of X1 on Y2.

In another instance, the disclosure features, a method for making a glycoprotein product. The method comprises:
a) optionally, providing a selected production parameter
b) providing a production system, e.g., a cell culture system, which incorporates a selected production parameter; and
c) maintaining said system under conditions which allow production of the glycoprotein product, thereby making the glycoprotein product, wherein the selected production parameter was identified by a method described herein, e.g., by:
   i) providing a database that correlates, defines, identifies, relates or provides each of a plurality of glycan properties as a correlative function of one or more production parameters or combinations of production parameters;
   ii) identifying a target glycan property, e.g., a glycan property of a primary glycoprotein product; and
   iii) selecting from the database one or more production parameters or combinations of production parameters that correlate with the target glycan property.

In an instance the selected production parameter was or is identified by:
selecting a primary glycoprotein,
providing a glycan pattern representing glycan structures on a reference glycoprotein, e.g., a primary glycoprotein, e.g., by releasing glycans from the reference glycoprotein, e.g., by enzymatic digestion, and optionally by separating the released glycans, e.g., to produce fractions or peaks representing one or more glycan properties,
selecting a glycan property,
selecting from the database one or more production parameters or combinations of production parameters that correlates with the target glycan property.

In an instance, providing a selected production parameter includes receiving the identity of the parameter from another entity. In an instance a first entity performs one or more of a), b) and c) and a second entity performs one or more of steps i), ii), and iii) and transmits the identity of the selected parameter to the first entity. Thus, as in other methods described herein, a single entity may perform all steps or may receive or by provided with information or selections needed to practice by one or more second entity.

In an instance the database includes ten or more, e.g., 20, 25, 50, 100, 150, 200, 300, 350, 400, 500, 600, 700, 800, 900 or more tunable, nonlinear, pleiotropic, or constrained correlations. In an instance a selected production parameter is associated with a tunable, nonlinear, pleiotropic, or constrained correlation.

In another instance, the disclosure features, a method of producing a glycoprotein product having one or a plurality of target glycan properties, including:
a) identifying a target glycan property or properties, e.g., a glycan property of a primary glycoprotein product; and
b) producing said glycoprotein product having one or a plurality of target glycan property or properties by a production method, wherein said production method was/is selected as follows:
   i) optionally characterizing a primary glycoprotein product so as to identify one or a plurality of glycan properties, e.g., glycan characteristics, of the primary glycoprotein product;
   ii) optionally, providing a database that correlates, defines, identifies, relates, or provides, each of a plurality of glycan properties as a correlative function of one or more production parameters or combinations of production parameters; and
   iii) selecting for use in the production method 1, 2, 3, or more production parameters, or combinations of production parameters, positively correlated with the incidence of said target glycan property or properties, e.g., selecting one or more production parameters or combinations of production parameters based on the correlations provided by said database.

In an instance the database includes ten or more, e.g., 20, 25, 50, 100, 150, 200, 300, 350, 400, 500, 600, 700, 800, 900 or more of a tunable, nonlinear, pleiotropic, or constrained correlation. In an instance a selected production parameter is associated with a tunable, nonlinear, pleiotropic, or constrained correlation.

In an instance the method further includes one or more of the following steps:
iv) expressing an amino acid sequence, preferably the amino acid sequence of said primary glycoprotein product, in a process using said selected parameter(s) and determining if the target glycan property, e.g., a glycan characteristic correlated with said selected parameter(s) is conferred on said amino acid sequence;
v) selecting an additional production parameter from said database;
vi) expressing an amino acid sequence, preferably the amino acid sequence of said primary glycoprotein product, in a process using said additional selected parameter and determining if the glycan property, e.g., glycan characteristic correlated with said additional selected parameter is included on said amino acid sequence; and
vii) optionally, repeating steps v and vi 1, 2, 3 or more times.

In another instance, the disclosure features, a method for making a glycoprotein product including making the glycoprotein by a process selected by:
i) identifying one or a plurality of required glycan properties, e.g., glycan characteristics, of said glycoprotein product;
ii) identifying one or more production parameters which will provide said one or plurality of required glycan properties; and
iii) sequentially selecting at least 2, 3, 4 or 5 production parameters to provide the required glycan property or characteristic, wherein said production parameters can be selected from the group consisting of: cell identity, cell culture conditions, fermentation conditions, isolation conditions, and formulation conditions, and combinations thereof.

In some instances methods described herein can be computer implemented. In other instances the methods are not computer implemented, e.g., a database relied on is not computer implemented. Instances can include displaying, outputting, or memorializing a selected production parameter or glycan characteristic.

### Methods of Designing Production Protocols

Methods disclosed herein allow for designing protocols or selecting conditions for making glycoproteins. The methods allow for the choice of production parameters, which when incorporated into a protocol for making a glycoprotein, provide for the incorporation into the glycoprotein of preselected glycan structures and/or glycan mediated functional properties.

In another instance, the disclosure features, a method, e.g., a computer-implemented method, including:
selecting a production parameter;
identifying a glycoprotein property, e.g., a glycoprotein characteristic, which is associated with said production parameter; and
optionally displaying, outputting, or memorializing said identified glycoprotein property.

In another instance, the disclosure features, a method, e.g., a computer-implemented method, including:
selecting a glycoprotein property, e.g., a glycoprotein characteristic;
identifying a production parameter which is associated with said glycoprotein property; and
optionally displaying, outputting, or memorializing said identified production parameter.

In another instance, the disclosure features, a method for designing a process to produce a glycoprotein product, or selecting an element of a process for making a glycoprotein product, the method including the steps of:
a) providing a database that correlates, defines, identifies, relates, or provides each of a plurality of glycan properties as a correlative function of one or more production parameters or combinations of production parameters;
b) identifying a target glycan property, e.g., a glycan property of a primary glycoprotein product;
c) selecting from the database one or more production parameters or combinations of production parameters that correlate with the target glycan property; and
thereby designing a process to produce a glycoprotein product.

In another instance, the disclosure features, a method of designing a process for making, or selecting an element of a process for making, a glycoprotein product the method including:
a) identifying a target glycan property or properties, e.g., a glycan property of a primary glycoprotein product; and
b) optionally characterizing the primary glycoprotein product so as to identify one or a plurality of glycan properties, e.g., glycan characteristics, of said primary glycoprotein product;
c) providing a database that correlates, defines, identifies, relates, or provides, each of a plurality of glycan properties as a correlative function of one or more production parameters or combinations of production parameters; and
d) selecting for use in the production method 1, 2, 3, or more production parameters, or combinations of production parameters, positively correlated with the incidence of said target glycan property or properties, e.g., selecting one or more production parameters or combinations of production parameters based on the correlations provided by said database, thereby designing a process for making, or selecting an element of a process for making, a glycoprotein product.

In another instance, the disclosure features, a method of designing a process for making, or selecting an element of a process for making, a glycoprotein product, the method including:
i) identifying one or a plurality of required glycan characteristics of said glycoprotein product;
ii) identifying one or more production parameters which will provide said one or plurality of required glycan characteristic; and
iii) sequentially selecting at least 2, 3, 4 or 5 production parameters to provide the required glycan characteristics, wherein said production parameters can be selected from the group consisting of: cell identity, cell culture conditions, fermentation conditions, isolation conditions, and formulation conditions, and combinations thereof.

In an instance of methods described herein the database can include one or more of a tunable, nonlinear, pleiotropic, or constrained correlation. In an instance a selected production parameter is associated with a tunable, nonlinear, pleiotropic, or constrained correlation.

In some instances methods described herein can be computer implemented. In other instances the methods are not computer implemented, e.g., a database relied on is not computer implemented. Instances can include displaying, outputting, or memorializing a selected production parameter or glycan characteristic.

### Control and Monitoring of Glycoprotein Production

Methods, databases and systems described herein can be used in a variety of applications, including methods of quality control or production monitoring. E.g., methods disclosed herein can be used to monitor a glycoprotein made by a defined process. E.g., if the glycoprotein is analyzed and found not to have a required glycan property methods described herein can be used to select alterations in the production process to tune or alter the process so that it produces a glycoprotein having the required glycan property.

Accordingly, the disclosure features, a method of monitoring and/or controlling the production of a glycoprotein. The method includes:
a) providing an observed glycan characteristic from a glycoprotein made by a predetermined production process;
b) providing a comparison of the observed glycan characteristic to a reference value;
c) if the observed value differs by more than a threshold level from the reference value selecting a value for a production parameter by a method described herein, e.g., by use of a database described herein; and
d) optionally altering the value of production parameter X in said predetermined production process to provide an altered production process,
thereby monitoring and/or controlling the production of a glycoprotein.

In an instance the method further includes the step of providing an observed glycan characteristic from a glycoprotein made by the altered production process and evaluating it as described herein. In instances, steps b), c) and d) are repeated for a glycoprotein made by the altered production process.

In an instance the method is repeated, e.g., at predetermined intervals.

In an instance selecting a value for a production parameter includes:
i) providing a database that correlates, defines, identifies, relates or provides each of a plurality of glycan properties as a correlative function of one or more production parameters or combinations of production parameters;
ii) optionally identifying a target glycan property; and
iii) selecting from the database one or more production parameters or combinations of production parameters which shifts the observed glycan property in the direction of the reference glycan property.

In an instance the observed glycan property was or is determined by:
providing a glycan pattern representing glycan structures on the glycoprotein made by the preselected production process, e.g., by releasing glycans from the glycoprotein, e.g., by enzymatic digestion, and optionally by separating the released glycans, e.g., to produce fractions or peaks representing one or more glycan property.

In an instance the reference glycan characteristic was or is determined by: providing a glycan pattern representing glycan structures on the glycoprotein made by the preselected production process, e.g., by a different, earlier run of the preselected process, or by a different production process, e.g., an altered production process, e.g., by releasing glycans from the glycoprotein, e.g., by enzymatic digestion, and optionally by separating the released glycans, e.g., to produce fractions or peaks representing one or more glycan characteristics.

In an instance the database includes one or more of at least a tunable, nonlinear, pleiotropic, or constrained correlation. In an instance a selected production parameter is associated with a tunable, nonlinear, pleiotropic, or constrained correlation.

### Databases

This section describes aspects and elements of databases of the disclosure. These can optionally be combined with methods and systems described herein.

Accordingly, in another instance, the disclosure features, a database described herein, e.g., a database useful in a method of system described herein.

In an instance the database is: disposed on tangible medium; disposed on a single unit of tangible medium, e.g., on a single computer, or in a single paper document; provided on more than one unit of tangible medium, e.g., on more than one computer, in more than a single paper document, partly on a paper document and partly on computer readable medium; disposed on computer readable medium; disposed on traditional medium, e.g., paper, which is readable by a human without the use of a computer, e.g., a printed document, chart, table or card catalogue.

In an instance: every element of the database is not stored in the same place, computer, memory or location; the database is configured to allow computerized access.

In an instance the database includes a plurality of records wherein a record includes,
an identifier for a production parameter or a combination of production parameters,
an identifier for a glycan property, e.g., a functional property conditioned by a glycan, or a glycan characteristic (i.e., a structural characteristic), and
a correlative function between the production parameter (or combination) and the glycan property, which e.g., correlates, defines, identifies, relates, or provides one to the other.

In an instance the correlation: is a positive or negative correlation; was or can be established by empirical testing or by prediction; is qualitative, e.g., positive, negative, or no correlation; is quantitative, e.g., a positive correlation can be expressed as a series of scores increasingly higher correlation; is expressed in absolute terms or as relative to a standard, e.g., as more or less, how much, more or less likely to confer a particular glycan characteristic on a protein, as another method.

### Systems

This section describes instances and elements of systems useful for implementing methods and databases described herein.

Accordingly, in another instance, the disclosure features, a system which includes:
a selector to select a production parameter based on an input glycoprotein property or to select a glycoprotein property based on an input production parameter.

In an instance the system includes:
a database described herein, e.g., a database that that correlates, defines, identifies, relates, or provides each of a plurality of glycan properties as a correlative function of one or more production parameters or combinations of production parameters;
a user interface for inputting a query;
a processor for generating a query result.

In an instance the system is configured to allow the design of a process to produce a target glycoprotein product having a preselected glycan property, e.g., to select a production parameter for the use in a method of producing a glycoprotein having a preselected glycan property.

In an instance said query is based on a selected glycan property, e.g., of a target glycoprotein product, and said query result includes one or more production parameters or combinations of production parameters from the database that correlate with the selected glycan property.

In an instance said query is based on one or more production parameters from the database that correlate with a selected glycan property and said query result is based on a glycan property correlated with said production parameter(s).

In an instance said user interface is configured to allow input of a desired glycan property and said processor is configured to allow output of a query result based on a correlated production parameter.

In an instance said user interface is configured to allow input of a desired production parameter and said processor is configured to allow output of a query result based on a correlated glycan property.

In an instance said system is configured to allow input of one or more values of X and output, e.g., a query result, of one or more values of Y, wherein a correlative function in said database relates X to Y, where X is a value for an element related to a production parameter and Y is a value for an element related to the glycan property, and said system is configured for adjustment of the value for X to select or identify a value for Y.

In an instance said system is configured to allow input of one or more values of Y and output of one or more values of X, wherein a correlative function in said database relates X to Y, where X is a value for an element related to a production parameter and Y is a value for an element related to the glycan property and the system is configured for adjustment of the value for Y to select or identify a value for X.

In an instance a production parameter 1 is tunable for an input setting (or value) X1 and the output or setting (or value) for Y1 will vary with the setting (or value) of X1, a production parameter 2 is tunable for an input setting (or value) X2 and the output or setting (or value) for Y2 will vary with the setting (or value) of X2.

In an instance some combination of values or settings for X1 and X2, or Y1 and Y2, are not compatible and the solution space, or total number of possibilities for the available combinations of Y1 and Y2, is less than the product of number of possibilities for Y1 and the number of possibilities for Y2 (or the analogous situation for X1X2).

In an instance a constraint on solution space is imposed by incompatibilities on combinations of X1 and X2, e.g., they may be concentrations of additives or combinations of additives and cells which cannot be combined for one reason or another.

In an instance a constraint on solution space is imposed because a combination of Y1 and Y2 are synthetically or structurally impossible or result in toxicity to the cell culture or to an unwanted property in a glycoprotein.

In an instance a correlative function produces a null output or a signal corresponding to an unavailable combination.

In an instance said system is configured with a filter which identifies prohibited or unavailable combinations of X1X2 or Y1Y2 and labels them or removes them from output.

In an instance a selection for a value for parameter X2 will be made based at least in part on the value chosen for X1.

In an instance the system is computer implemented.

In an instance the system is not computer implemented.

In an instance the system includes a correlative function which is a tunable, nonlinear, pleiotropic, or constrained correlation.

### Correlative Functions

Some of the methods, systems and databases described herein feature correlative functions. The following section provides additional details, specific instances and alternatives for correlative functions. These are not limiting but are rather exemplary. They can optionally be incorporated into methods, databases, or systems described herein.

### Tunable Correlative Functions

A tunable function can allow for a plurality of inputs, e.g., inputs of differing magnitudes, and a plurality of outputs, e.g., of differing magnitude. It can allow the adjustment of a glycan property by the adjustment of a production parameter. Thus, in an instance, a correlative function is a tunable function. By way of example, a correlative function relates X to Y, where X is a value for an element related to a production parameter and Y is a value for an element related to the glycan property and allows adjustment of the value for X to select or identify a value for Y or the adjustment of the value for Y to select or identify a value for X. By way of example, X can be any of a value for concentration of an additive, a value of a byproduct, a value of a physical parameter, a value of time, a value of cell type, a value of gene expression level of copy number, and, in one or more of those cases, Y the amount of a glycan structure on a glycoprotein.

In an instance, a production parameter 1 is tunable for an input setting (or value) X1 and the output or setting (or value) for Y1 will vary with the setting (or value) of X1, a production parameter 2 is tunable for an input setting (or value) X2 and the output or setting (or value) for Y2 will vary with the setting (or value) of X2. In some instances, some combination of values or settings for X1 and X2, or Y1 and Y2, are not compatible and the solution space, or total number of possibilities for the available combinations of Y1 and Y2, is less than the product of number of possibilities for Y1 and the number of possibilities for Y2 (or the analogous situation for X1X2).

In some instances a constraint on solution space imposed by incompatibilities on combinations of X1 and X2, e.g., they may be concentrations of additives or combinations of additives and cells which cannot be combined for one reason or another.

In some instances a constraint on solution space is imposed because a combination of Y1 and Y2 are synthetically or structurally impossible or result in toxicity to the cell culture or to an unwanted property in a glycoprotein. In some instances a correlative function produces a null output or a signal corresponding to an unavailable combination.

### Nonlinear Correlative Functions

A nonlinear correlation can reflect a relationship between production parameters and glycan properties wherein the effect of two (or more) production parameters acting together on a glycan property is not the same as the combination of the first production parameter (acting alone) on the glycan property together with the effect of the second production parameter (acting alone) on the glycan property. This can be expressed as "X1,X2-Y1 ≠ X1-Y1 + X2-Y1. in the notation used herein.

In some instances a correlative function relates values for more than one value for production parameters (e.g., X1, X2, and so on) to one or more glycan property, e.g., Y, and wherein the effect of the combination, e.g., the combination of X1 and X2, on Y is nonlinear. The correlation is nonlinear when the effect of a plurality of production parameters acting together, e.g., production parameters X1 and X2 (acting together), on one or more glycan properties, e.g., Y, is not the same as the combination of X1 (acting alone) on Y together with the effect of X2 (acting alone) on Y. By way of example, the addition of glucosamine (X1) results in a decrease in galactosylation (Y1), a decrease in fucosylation (Y2), an increase in high mannose structures (Y3), and an increase in hybrid structures (Y4). The addition of uridine (X2) gives a decreases high mannose structures (Y3) but no change of the other glycan properties (Y1, Y2, and Y4). If glucosamine (X1) and uridine (X2) are combined all four parameters, Y1, Y2, Y3 and Y4, are unchanged. Thus, the correlative function between X1,X2 and Y1 is nonlinear. Likewise, the correlative function between X1, X2 and Y2 is nonlinear and the correlative function between X1, X2 and Y4 is nonlinear. In some instances single X correlations, which are nonlinear when taken together, are also considered, individually, to be nonlinear. E.g., in the example just given, the correlation of glucosamine (X1) with galactosylation (Y1), the correlation of glucosamine (X1) with fucosylation (Y2) and the correlation of glucosamine (X1) with hybrid structures (Y4) are all nonlinear. Similarly, the correlation between uridine (X2) with galactosylation (Y1), the correlation ofuridine (X2) with fucosylation (Y2) and the correlation of uridine (X2) with hybrid structures (Y4) are considered nonlinear correlations in some instances.

### Constrained Correlative Functions

Constrained correlative functions reflect the complexity of glycoprotein synthesis and can represent relationships characterized by incompatible or undesirable combinations or production parameters or glycan properties. E.g., a combination of production parameters may be constrained because it results in an undesirable glycan property. In some instances a correlative function relates a value for a first production parameter X1 to a first to a value for a first glycan property Y1 but also identifies either or both of: an additional, e.g., second, glycan property Y2 which is altered by X1; and an additional, e.g., second, production parameter X2 which can be used along with the first production parameter, e.g., to modulate, e.g., minimize, the overall effect on a second glycan property Y2. This correlation is referred to as a constrained production parameter, because the use of X1 may require the use of X2 as well to avoid an unwanted affect on glycan property Y2. In instances the selection of a first production parameter may constrain the selection of a second production parameter and makes the selection of a specific second production parameter more or less favored, because, e.g., of a positive or negative effect on the conferral of a glycan property on the protein if the second parameter is (or is not) combined with the first. By way of example, the addition of glucosamine, X1, is correlated with a decrease in galactosylation. X1 is also correlated with an increase in high mannose. The addition of uridine, X2, minimizes the increase in high mannose without abolishing the X1 mediated decrease in galactosylation. If a decrease in galactose is desired but an increase in high mannose is not desired then X1 is constrained. The X1-Y1 correlation or an X1-Y1,Y2 correlation can identify X2 as an additional production parameter to be considered or altered in conjunction with X1.

### Pleiotropic Correlative Functions

Pleiotropic correlative functions can reflect the varied effect of one or more production parameters on different glycan characteristics. In some instances a correlative function relates X to a plurality of glycan properties, and the relationship is pleiotropic. E.g., where X is a value for an element related to a production parameter and Y1 and Y2 (and optionally Y3, Y4, Y5, and so on) are each values for elements related to the glycan properties, production parameter X confers different effects (in an instance these effects are in different directions, e.g., one is increased and the other is decreased, as opposed to one is changed, e.g., increased or decreased, and the other is unchanged) on at least two glycan properties. By way of example, production parameter X, the addition of glucosamine to the media, is correlated with a reduction in galactosylation (e.g., Y1), reduction in fucosylation (e.g., Y2), an increase in high mannose (e.g., Y3) and an increase in hybrid structures (e.g., Y4).

### Glycoprotein Analysis

Some of the methods, systems and databases described herein include or relate to additional steps, e.g., steps in which a glycoprotein product is further analyzed. Some specific preferred instances of these methods, systems and databases are provided below.

In an instance a method further includes analyzing an amino acid sequence, e.g., that of the primary glycoprotein product, produced under said selected combination of production parameters and comparing it with a preselected criterion, e.g., the presence, absence or level of a preselected glycan property, e.g., glycan characteristic. E.g., if the amino acid sequence has a preselected relationship with the criterion, e.g., it meets or fails to meet said criteria, selecting the combination. In an instance a method further includes altering the conditions of the selected combinations, e.g., by altering the growth medium, based on whether the glycoprotein exhibits the preselected relationship.

In an instance a method further includes analyzing the glycoprotein produced under a selected combination and comparing it with a preselected criterion, e.g., having a preselected glycan property, e.g., a glycan structure. If, e.g., the glycoprotein has a preselected relationship with said preselected criteria, e.g., it meets or fails to meet said criteria, the method includes selecting the combination or glycoprotein produced by the combination for further analysis, e.g., alteration of another parameter, e.g., altering the growth medium.

In an instance a method further includes testing the glycoprotein product made by the production method to see if it has a preselected chemical, biological, or pharmacokinetic, property. E.g., the method can include comparing a preselected chemical, biological, or pharmacokinetic or pharmacodynamic, property of the glycoprotein made by the production process with a preselected standard and if the value for said glycoprotein product has a preselected relationship with the preselected standard selecting said glycoprotein product.

In an instance a property of a glycoprotein product is compared with a property of a primary glycoprotein product.

Instances of methods described herein include analyzing a glycoprotein product, e.g., a primary glycoprotein product for glycan properties, e.g., glycan characteristics. This analysis can be used as a guide for selecting production parameters or in producing a glycoprotein. The analysis can be based on information produced by releasing glycan structures from the glycoprotein. In this context release means release from all or at least some of the amino acid portion of the glycoprotein. By way of example, the method can use complete or partial enzymatic digestion to release glycan structures, e.g., as single saccharides or larger fragments, from a glycoprotein. The released glycan structures can be analyzed, e.g., by providing a glycan pattern and comparing it to a predetermined standard, e.g., a reference glycan pattern. A glycan pattern, as used herein, is a representation of the presence (or absence) of one or more glycan properties. In instances the glycan pattern provides a quantitative determination of one or more glycan properties. The quantitative determination can be expressed in absolute terms or as function of a standard, e.g., an exogenous standard or as a function of another glycan property in the pattern. The elements of a glycan pattern can, by way of example, be peaks or other fractions (representing one or more species) from a glycan structures derived from a glycoprotein, e.g., from an enzymatic digest. Elements can be described, e.g., in defined structural terms, e.g., by chemical name, or by a functional or physical property, e.g., by molecular weight or by a parameter related to purification or separation, e.g., retention time on a column or other separation device. Methods described herein can be used to make a glycoprotein having desired glycan properties. This includes the design of a process to make such a glycoprotein or its production. The analysis can be used to determine if or confirm that a glycoprotein has selected glycoprotein properties. By way of example, methods described herein can be used to monitor production processes and to select production parameters to refine a process which produces product which fails to meet a standard, e.g., does not posses a selected glycan property.

In an instance a method further includes selecting said glycoprotein product for, classification, acceptance or discarding, releasing or withholding, processing into a drug product, shipping, being moved to a new location, formulation, labeling, packaging, releasing into commerce, for being sold, or offered for sale, or submission if information about the glycoprotein product to a third party for review or approval, depending on whether the preselected criterion is met.

In an instance, at the time of designing or using the production method, the designer or user has searched, e.g., by consulting a government or commercial listing of patents, for the existence of a U.S. patent which covers the reference glycoprotein product, or a method of making or using the reference glycoprotein product.

In an instance, a method further includes a step, e.g. before step ii of a method herein, of analyzing a target glycoprotein product to identify a target glycan property.

In an instance, a method further includes expressing the amino acid sequence of said primary glycoprotein product under said selected condition or conditions and determining if the selected condition or conditions is positively correlated with the presence of the target glycan-conditioned property in the glycoprotein.

Any of the methods described herein can include one or more of the following steps:
evaluating the glycoprotein product, e.g., evaluating physiochemical parameters of the glycoprotein product, e.g., measuring mass (e.g., using SDS-PAGE or size exclusion chromatography), pI, carbohydrate content, peptide mapping, protein concentration, biological activity of the glycoprotein product;
recording the evaluation of one or more parameters of the glycoprotein product, e.g., providing a certificate of analysis for the glycoprotein product;
assessing process contaminants of the glycoprotein or its cell culture, e.g., including but not limited to endotoxin content, sterility testing, mycoplasma content, leachates, host (e.g. CHO) cell DNA or protein contaminants;
recording the process contaminants of the glycoprotein or its cell culture;
measuring the glycoprotein cell culture process parameters, including but not limited to the production pH, cell viability, production, titer, yield, doubling time, DO, and temperature;
recording the cell culture process parameters;
assessing and recording the process media components, including raw materials source and lot numbers of materials;
measuring the glycoprotein purification process parameters, including but not limited to the flow rate, pH, temperature, yield, process contaminants, column volume, or elution volume;
recording the purification process parameters; and
recording a lot number of a glycoprotein batch made from a process described herein.

### Selection of Production Parameters and Glycan Properties

Some of the methods, systems and databases described herein include or relate to the selection, or the use, of a glycan property or a production parameter. Some specific instances of these methods, systems and databases are provided below.

In an instance, e.g., in step iii of a method herein, a plurality of production parameters or combination thereof are selected sequentially.

In an instance, e.g., in step ii of a method herein, the method includes identifying at least 2, 3, 4, or 5, target glycan properties.

In an instance, e.g., in step iii of a method herein, the method includes selecting a combination of production parameters, which combination correlates with a target glycan property.

In an instance, e.g., in a combination of at least 1 or 2 primary production parameters or at least 1 or 2 secondary parameters or at least one primary and one secondary parameter is selected.

In an instance a production parameter is selected to confer a target glycan property, e.g., a functional property, which differs from the corresponding glycan property of a primary glycoprotein product.

In an instance a glycan property is a functional property of a glycoprotein, e.g., serum half life, receptor binding affinity, or immunogenicity (in an instance it is other than immunogenicity).

In an instance, a glycan property is a glycan characteristic, i.e., a structural property. Exemplary glycan characteristics include: the presence, absence or amount of a chemical unit; the presence, absence or amount of a component of a chemical unit (e.g., a sulfate, a phosphate, acetate); heterogeneity or microheterogenity at a potential glycosylation site or across the entire protein, e.g., the degree of occupancy of potential glycosylation sites of a protein (e.g., the degree of occupancy of the same potential glycosylation site between two or more of the particular protein backbones in a glycoprotein product and the degree of occupancy of one potential glycosylation site on a protein backbone relative to a different potential glycosylation site on the same protein backbone); the core structure of a branched (e.g., the presence, absence or amount of bisecting GlcNAc phosphomannose structures) or unbranched glycan; the presence, absence or amount of a glycan structure (e.g., a complex (e.g., biantennary, triantennary, tetrantennary, etc.), a high mannose or a hybrid glycan structure); the relative position of a chemical unit within a glycan (e.g., the presence, absence or amount of a terminal or penultimate chemical unit); and the relationship between chemical units (e.g., linkages between chemical units, isomers and branch points).

In an instance a target glycan property is selected from the group consisting of: galactosylation, fucosylation, high mannose, sialylation, and combinations thereof.

In an instance at least 1, 2, 3, 4 or more production parameters are selected sequentially, e.g., each is selected on the basis of a correlation between a single production parameter and a glycan characteristic.

In an instance, between the selection of a first production parameter and the selection of a second production parameter the first production parameter is tested for the ability confer a selected glycan property (e.g., a glycan characteristic correlated with the first production parameter by the database) on an amino acid sequence, e.g., the amino acid sequence of the primary glycoprotein product.

In preferred instance, between the selection of a second production parameter a third production parameter the second production parameter is tested for the ability confer a selected glycan property (e.g., a glycan characteristic correlated with the second production parameter by the database) on an amino acid sequence, e.g., the amino acid sequence of the primary glycoprotein product.

In an instance 2, 3, 4 or more production parameters are selected simultaneously, e.g., a combination of production parameters is selected on the basis of a correlation between the combination of production parameters and a glycan property, e.g., a glycan characteristic.

In an instance, a method includes, e.g., in step iii:
selecting, in a sequential manner,
   i) a first production parameter, e.g., a primary production parameter, e.g., a parameter related to a cell line, a process or bioreactor variable, e.g., batch, fed-batch, or perfusion, a purification process or a formulation, from said database, said database including a correlation between said first production parameter and the conferral of a selected glycan property, e.g., a glycan characteristic, on a protein made in a process which includes said first production parameter; and
   ii) a second production parameter, e.g., a secondary production parameter, from said database, said database including a correlation between said secondary production parameter and the conferral of a selected glycan property, e.g., a glycan characteristic, on a protein made in a process which includes said second production parameter.

In an instance a method includes selection of 1, 2, 3 or more primary production parameters is interspersed with or followed by selection of 1, 2, 3 or more secondary production parameters.

In an instance a step in the production method is determined by selecting a production parameter which is correlated with the production of glycoprotein having said preselected glycan property, e.g., a glycan characteristic, from a database.

In an instance a step in the production method is determined by selecting a production parameter from a database in which each of a plurality of production parameters, or combinations of production parameters, e.g., at least 2, 5, 10, 20, 30, 40 or more parameters or combinations or parameters, is correlated with the production of glycoprotein having said preselected glycan property, e.g., a glycan characteristic, when said parameter or combination of parameters is incorporated into a method for making the glycoprotein product.

In an instance a production parameter is selected to confer a target glycan property, e.g., a functional property, which is the same as or similar to the corresponding glycan property of said primary glycoprotein product.

In an instance the production method is different from a published method for making said primary glycoprotein product.

In an instance a production parameter is selected which is correlated with the conferral on an amino acid sequence of a glycan characteristic, found on the glycoprotein product or is correlated with a glycan characteristic, which is an intermediate and which is positively correlated with the (eventual) presence on the expressed glycoprotein product of a selected glycan characteristic.

In an instance a production parameter is selected to confer a target glycan property, e.g., a functional property, which differs from the corresponding glycan property of said primary glycoprotein product.

In an instance a method includes selecting the glycan properties required by the glycoprotein product and then selecting the production parameters, e.g., those selected in d) of a method described herein, to provide the required glycan properties.

In an instance a method includes selecting a combination of production parameters, which combination correlates with a target glycan property.

### Exemplary Glycoproteins and Properties

Some of the methods, systems and databases described herein include or relate to an improved glycoprotein product, the selection of a method to make an improved glycoprotein product, or a method of making an improved glycoprotein product. Some specific preferred instances of these methods, systems and databases are provided below.

In an instance the glycoprotein product is an altered (or next generation) glycoprotein product having a preselected glycan property and wherein step b) includes:
selecting one or a plurality of glycan properties as said target glycan property(s) and wherein said target glycan property(s) is different from the corresponding glycan property(s) of said primary glycoprotein product, e.g., they differ in affinity for a receptor or the degree of heterogeneity of glycan structures attached at a preselected site.

In an instance the production method results in a glycoprotein product having different glycan characteristic(s) than said primary glycoprotein target.

In an instance the target glycan property is serum half life which is longer or shorter than the serum half life of the primary glycoprotein product.

In an instance a target glycan property is serum half life which is longer or shorter than the serum half life of the primary glycoprotein product.

Some of the methods, systems and databases described herein include or relate to the analysis of a primary glycoprotein product. Some specific preferred instances of these methods, systems and databases are provided below.

In an instance a method includes providing information resulting from subjecting the primary glycoprotein product to one or more of the analytical method described herein to provide a glycan property, e.g., a glycan characteristic. The analytical method can be applied to one or a plurality of samples of the primary glycoprotein product, e.g., commercially available primary glycoprotein product. The analytical method can be applied to one or a plurality of production lots of the primary glycoprotein product, e.g., commercially available primary glycoprotein product.

In an instance the primary glycoprotein product and the glycoprotein product have identical amino acid sequences.

In an instance the primary glycoprotein product and the glycoprotein product differ by up to 1, 2, 3, 4, 5, 10 or 20 amino acid residues.

In an instance the primary glycoprotein product is selected from Table 1.

### Databases: Additional Instances

Databases, and methods and systems which include the use of a database, are described herein. Some specific preferred instances of these databases are provided below.

In preferred instances a database has at least 5, 10, 20, 30, 40, 50, 100, 150, 200, 250 correlations records.

In an instance a database provides:
a correlation between a first production parameter (or combination of production parameters) and the conferral of a selected glycan property, e.g., glycan characteristic, on a protein made by a process which includes said first parameter;
a correlation between a second production parameter (or combination of production parameters) and the conferral of said selected glycan property, e.g., glycan characteristic, on a protein made by a process which includes said second production parameter; and
the database is configured so as to allow choice between the first and second parameter.

In an instance a database provides:
a correlation between the use of the combination of a first and second production parameter (or respective combinations) in a process for making said glycoprotein product on the conferral of said selected glycan characteristic on a protein made by said combination process;
and allows the provision of information on the effect of the addition of the first or second production parameter (or respective combinations) on the other production parameter (or respective combination) in terms of addition of a selected glycan property, e.g., glycan characteristic, to a protein.

In an instance a database is configured so as to allow making a decision of whether to include the first, second, or both production parameters in the production of a glycoprotein product.

In an instance a database is configured to allow appreciation that selection of a first production parameter constrains the selection of a second production parameter and makes the selection of a specific second production parameter more or less favored, because, e.g., of a positive or negative affect on the conferral of a glycan property on the protein if the second parameter is combined with the first.

In an instance the database includes one, two, three, or all of a tunable, nonlinear, pleiotropic, or constrained correlation.

In an instance a database includes:
i) a correlation between a first and second production parameter and conferral of a first selected glycan characteristic on a protein made by a method which includes said first and second (but not a third) production parameter
ii) a correlation between said first and third production parameters and conferral of said first selected glycan characteristic on a protein made by a method which includes said first and third (but not said second) production parameter; and allows comparison of (1) the presence, on a protein made by a method which includes the first and second (but not said third) production parameter, of a first selected glycan characteristic with (2) the presence, on a protein made by a method which includes the first and the third but not the second production parameter,
   and further allows a choice between the combination of i and the combination of ii on the grounds of optimization of the presence of said first selected glycan characteristic.

In an instance a database includes:
correlations each of a plurality of species of a first generic production parameter, e.g., variants of a cell type, e.g., a plurality of CHO cells having different sites of insertion, copy number of insertion, or glycoslyation-related genes with the conferral of a of a glycan property, e.g., a glycan characteristic, on a protein made by a method which includes use of the species; and
correlations each of a plurality of species of a second generic production parameter, e.g., fermentation variants, e.g., a plurality of fermentation conditions such as, cell density, batch process, perfusion process, continuous process with the conferral of a of a glycan property, e.g., a glycan characteristic, on a protein made by a method which includes use of the species.

In an instance a database includes:
a correlation between the combination of a first species of a first production parameter and a first species of a second production parameter with the conferral of a glycan property, e.g., a glycan characteristic, on a protein made by a method which includes combination;
a correlation between the combination of a second species of a first production parameter and the first or a second species of a second production parameter with the conferral of a of a glycan property, e.g., a glycan characteristic, on a protein made by a method which includes combination
and allows comparison (and choice) between (1) a combination of first species of a first generic production parameter, e.g., a CHO cell having insertion at a first site, and a first species of a second generic production parameter, e.g., batch-process fermentation, and (2) a combination of a different species of the first generic production parameter, e.g., a CHO cell having insertion at a second site, and a species of the said second generic production parameter, e.g., continuous process fermentation.

In an instance a database includes:
a correlation between a combination of production parameters and the conferral of a selected glycan property, e.g., a glycan characteristic, on a protein made in a process which includes said combination of production parameters, e.g., wherein the combination of production parameters includes a cell and a culture medium.

In an instance a database includes:
correlations between a cell cultured under each of a plurality of culture conditions, e.g., said cell cultured in each of a first, second, and third medium, and the conferral of a selected glycan property, e.g., a glycan characteristic, on a protein made in a process which includes said cell cultured under one of said culture conditions, e.g., wherein a selected cell type, e.g., a CHO cell, can be cultured in a plurality of media and each cell/condition combination correlated to the incidence of the same or a different glycan property, e.g., a glycan characteristic.

In an instance a database includes:
correlations between each of a plurality of cells cultured under a plurality of conditions, e.g., a first cell type cultured in a first, second, and third medium, a second cell type cultured in the first, second, and third medium, and a third cell type cultured in the first, second, and third medium, and the conferral of a selected glycan property, e.g., glycan characteristic, on a protein made in a process which includes a cell/condition combination.

In an instance a database includes a correlation between each of several selected cell types, e.g., different strains or genotype CHO cells, cultured in a plurality of media (cell/condition combinations) to the incidence of a glycan property, e.g., a glycan characteristic.

### Exemplary Glycoprotein Products

In another instance, the disclosure features, a glycoprotein product or preparation, e.g., a pharmaceutical preparation, of a glycoprotein product, made by a process described herein, e.g., a process of making a glycoprotein or a process of selecting the steps of a method for making a glycoprotein.

In an instance the glycoprotein product has the amino acid sequence of a protein from Table I, or differs by no more than 1, 2, 3, 4, or 5 amino acid residues from the protein of Table I.

In an instance the glycoprotein product differs by at least one glycan characteristic from the protein of Table I.

In another instance, the disclosure features, a glycoprotein product or preparation, e.g., a pharmaceutical preparation, of a glycoprotein product, having the amino acid sequence of a protein from Table I, or differs by no more than 1, 2, 3, 4, or 5 amino acid residues from the protein of Table I, wherein said glycoprotein product differs by one or more glycan characteristic listed in Table II from a commercial preparation of said protein.

In an instance the glycoprotein product or preparation, e.g., a pharmaceutical preparation, of a glycoprotein product, has one or more of: more or less fucosylation, more or less galactosylation, more or less high mannose structure, more or less hybrid structure, more or less sialylations, than does the corresponding protein from Table I.

In another instance, the disclosure features, a method of producing a protein with a modulated amount of a glycan characteristic selected from Table II by modulating a production parameter from Table II including:
selecting a reference level of said glycan characteristic, e.g., the level found on a preselected glycoprotein, e.g., a target glycoprotein;
selecting a value for a parameter from Table II to provide a modulated level of said glycan characteristic (as compared, e.g., to the reference level); and
applying the selected parameter in a process for making protein with a modulated amount of said glycan characteristic.

In another instance, the disclosure features, a method of producing a protein having a preselected level of a functional or biological property from Table III by modulating a parameter from Table II including:
selecting a reference level of said biological property, e.g., the level found on a preselected glycoprotein;
selecting a value for a parameter from Table II to provide a modulated level of said glycan characteristic which modulates said biological property;
   applying the selected parameter in a process for making protein with a modulated amount of said functional or biological property.

### Additional Instances of the Disclosure

In another instance, the disclosure features, a computer program product tangibly embodied in an information carrier and including instructions that when executed by a processor perform a method described herein.

Methods, databases, and systems described herein can be used with a wide variety of glycoproteins (including glycopeptides). These include naturally occurring and nonnaturally occurring glycoproteins. Representative glycoproteins include: antibodies, e.g., IgG, IgM, human, humanized, grafted, and chimeric antibodies, and fragments thereof; fusion proteins, e.g., fusions including human (or other) antibody domains, e.g., Fc or constant region domains; growth factors; hormones; and any class of protein represented by a protein listed in Table 1.

The term "database" as used herein, refers to a collection of data. Typically it is organized so that its contents can easily be accessed, managed, and updated. In preferred instances the database is configured or managed to ensure its integrity and quality, to minimize content beyond records described herein, and to allow controlled access. The database is presented or memorialized on a medium. The medium can be, e.g., a traditional paper medium or other medium which displays printed or written symbols which can be directly (e.g., without the aid of a computer) used by a human being. Such a database can exist as a set of printed tables, or a card catalogue, which, e.g., show the relationship of production parameters to glycan characteristics. The database can also be presented or memorialized in electronic or other computer readable form. Such databases can range from simple spreadsheets to more complex forms. The database need not be deposited on a single unit of medium, e.g., in a single table or book, or on a single computer or network. A database, e.g., can combine a traditional medium as described above with a computer-readable medium. Typically, the database will contain a collection of records, wherein each record relates a production parameter to a glycan property by way of a correlative function. The database can be organized in a number of ways, e.g., as a relational database. Typically the database is in a format that can be searched for specific information or records by techniques specific to each database. A computer database is typically a structured collection of records stored in a computer or computers so that a program can consult it to answer queries. Relational databases together with interfaces for queries and query results are particularly preferred. Mapping the ontology of a relational database allows building of correlations useful in the methods described herein.

While some instances of the disclosure may retrieve information from publicly accessible information, databases used in such instances will generally also include at least 1, 2, 5, 10, 20 or 50 correlations which are were not present in, or which were not retrieved from, publicly accessible information, e.g., in such instances the database may contain at least 1, 2, 5, 10, 20, 50 or more non-linear, pleiotropic, or constrained correlations. Publicly accessible information can include information from a publicly accessible database such as PubMed. In an instance a database described herein contains at least 1, 2, 5, 10, 20 or 50 correlations which are not in publicly accessible information, e.g., are not in published documents. The determination of whether a correlation is publicly accessible, e.g., in a published document or database, is made as of the earliest U.S. filing date of a nonprovisional application from which this patent claims priority.

The headings used in this document are for ease of reading and should not be used to limit the instances of the disclosure, e.g. embodiments of the invention, described.

### BRIEF DESCRIPTION OF THE DRAWINGS

The drawings are now described.
FIG. 1 is a block diagram of computing devices and systems.
FIG. 2 is a depiction of a representative chromatogram of glycan patterns from human IgG produced in CHO cells. Human IgG was produced from CHO cells, isolated, glycans released, isolated, and fluorescently tagged, prior to resolving on NP-HPLC.
FIG. 3 is a depiction of glycan patterns from human IgG produced in CHO cells under distinct process conditions. Human IgG was produced from CHO cells cultured in the presence of elevated uridine, glucosamine, or both. The IgG was isolated, glycans released, isolated, and fluorescently tagged, prior to resolving on NP-HPLC. A summation of the normalized data for the IgG produced in the presence of elevated uridine, glucosamine, or both is shown as indicated. Data are representative of duplicate determinants and are expressed as a % of the total peak area.

Other features and advantages of the invention and the disclosure will be apparent from the following detailed description, and from the claims.

### DETAILED DESCRIPTION

### Glycan properties:

Methods described herein include selecting one or more production parameters to produce a glycoprotein having one or more preselected glycan properties. A glycan property, as used herein, refers to (1) a functional property conferred or conditioned by a glycan structure on a protein or (2) a structural property (referred to herein as a "glycan characteristic").

A preselected functional activity can be correlated with a glycan characteristic or characteristics and based upon that correlation a decision can be made regarding which production parameter or combination of production parameters result in a glycoprotein having the preselected glycan characteristic and, thus, functional activity. Activities that can be selected include, but are not limited to, serum half life, clearance, stability *in vitro* (shelf life) or *in vivo,* binding affinity, tissue distribution and targeting, toxicity, immunogenecity, absorption rate, elimination rate, three dimensional structure, metabolism and bioavailability.

A "glycan characteristic" as used herein includes the presence, absence or amount of a chemical unit; the presence, absence or amount of a component of a chemical unit (e.g., a sulfate, a phosphate, an acetate, a glycolyl, a propyl, and any other alkyl group modification); heterogeneity or microheterogenity at a potential glycosylation site or across the entire protein, e.g., the degree of occupancy of potential glycosylation sites of a protein (e.g., the degree of occupancy of the same potential glycosylation site between two or more of the particular protein backbones in a glycoprotein product and the degree of occupancy of one potential glycosylation site on a protein backbone relative to a different potential glycosylation site on the same protein backbone); the structure of a branched (e.g., the presence, absence or amount of bisecting GlcNAc or phosphomannose structures) or unbranched glycan; the presence, absence or amount of a glycan structure (e.g., a complex (e.g., biantennary, triantennary, tetrantennary, etc.), a high mannose or a hybrid glycan structure); the relative position of a chemical unit within a glycan (e.g., the presence, absence or amount of a terminal or penultimate chemical unit); the chemical makeup of the glycan (e.g. amounts and ratios of the monosaccharide components in a particular glycan); and the relationship between chemical units (e.g., linkages between chemical units, isomers and branch points). In instances a glycan characteristic can, by way of example, be a peak or other fraction (representing one or more species) from glycan structures derived from a glycoprotein, e.g., from an enzymatic digest. A glycan characteristic can be described, e.g., in defined structural terms, e.g., by chemical name, or by a functional or physical property, e.g., by molecular weight or by a parameter related to purification or separation, e.g., retention time of a peak in a column or other separation device.

A "chemical unit" as used herein is a chemical compound of carbon, hydrogen and oxygen in which the atoms of the later two elements are in a ratio of 2:1. A chemical unit can be, e.g., an aldehyde or ketone derivative of a polyhydric alcohol, particularly pantahydric and hexahydric alcohols. Examples of chemical units include monosaccharides such as galactose, fucose, sialic acid, mannose, glucose, N-acetylglucosamine (GlcNAc), N-acetylgalactosamine (GalNAc) and ribose, as well as derivatives and analogs thereof. Derivatives of various monosaccharides are known. For example, sialic acid encompasses over thirty derivatives with N-acetylneuraminic acid and N-glycolylneuraminic acid forming the core structures. Synthetic ganglioside derivatives are described in U.S. Patent No. 5,567,684; bivalent sialyl-derivatized saccharides are described in U.S. Patent No. 5,559,103. Derivatives and analogues of 2-deoxy-2,3-didehydro-N acetyl neuraminic acid are described in U.S. Patent No. 5,360,817. Examples of sialic acid analogs include those that functionally mimic sialic acid, but are not recognized by endogenous host cell sialylases. Sialyltransferases and other enzymes that are involved in sialic acid metabolism often recognize "unnatural" or "modified" monosaccharide substrates (Kosa et al., Biochem. Biophys. Res. Commun., 190, 914, 1993; Fitz and Wong, J., Org. Chem., 59, 8279, 1994; Shames et al., Glycobiology, 1, 187, 1991; Sparks et al., Tetrahedron, 49, 1, 1993; Lin et al., J. Am. Chem. Soc., 114, 10138, 1992). Other examples of monosaccharide analogs include, but are not limited to, N-levulinoyl mannosamine (ManLev), Neu5Acα-methyl glycoside, Neu5Acβ-methyl glycoside, Neu5Acα-benzyl glycoside, Neu5Acβ-benzyl glycoside, Neu5Acα-methylglycoside methyl ester, Neu5Acα-methyl ester, 9-O-Acetyl-N-acetylneuraminic acid, 9-O-Lactyl-N-acetylneuraminic acid, N-azidoacetylmannosamine and O-acetylated variations thereof, and Neu5Acα-ethyl thioglycoside. In addition, examples of sialic acid analogs and methods that may be used to produce such analogs are taught in U.S. Patent No. 5,759,823 and U.S. Patent No. 5,712,254.

Examples of derivatives, or analogs, of other monosaccharides include: amidine, amidrazone and amidoxime derivatives of monosaccharides (U.S. Patent No. 5,663,355), 1,3,4,6-tetra-O-acetyl-N-acylmannosamine or derivative thereof, analogs or derivatives of sugars or amino sugars having 5 or 6 carbons in the glycosyl ring, including aldoses, deoxyaldoses and ketoses without regard for orientation or configuration of the bonds of the asymmetric carbons. This includes chemical units such as ribose, arabinose, xylose, lyxose, allose, altrose, glucose, idose, galactose, talose, ribulose, xylulose, psicose, N-acetylglucosamine, N-acetylgalactosamine, N-acetylmannosamine, N-acetylneuraminic acid, fructose, sorbose, tagatose, rhamnose and fucose. Exemplary monosaccharide analogs and derivatives derived from glucose, N-acetylglucosamine, galactose, N-acetylgalactosamine, mannose, fucose and sialic acid as taught, for example in U.S. Patent No. 5,759,823.

A glycan characteristic can include the presence, absence or amount of various derivatives or analogs of a chemical unit. For example, the glycan characteristic can be the absence, presence or amount of N-acetyl neuraminic acid.

A "glycan structure" as used herein refers to at least two chemical units linked to one another. Any linkage, including covalent and non-covalent linkages, is included.

A glycan characteristic can further be a comparison of the presence, absence or amount of a chemical unit, a component of a chemical unit or a glycan structure relative to the presence, absence or amount of another chemical unit, another component of a chemical unit or another glycan structure, respectively. For example, the presence, absence or amount of sialic acid relative to the presence absence or amount of fucose can be determined. In other examples, the presence, absence or amount of a sialic acid such as N-acetylneuraminic acid can be compared, e.g., to the absence, presence or amount of a sialic acid derivative such as N-glycolylneuraminic acid.

A correlative function as used herein provides a function which defines the relationship, e.g., in a database, between one or more production parameters and one or more glycan properties. In an instance, one production parameter is correlated to one glycan property. The correlative function can embody a constant value, e.g., a positive constant value in the case of a positive correlation between the presence or use of a production parameter and the conferral of a glycan property on a glycoprotein. Instances also include those in which a plurality of species of the production parameter (e.g., different concentrations of a specified additive) are each assigned a different correlative constant each having a different constant value. E.g., in the case of a production parameter such as glucosamine, which can be added to culture conditions at different concentrations, and the glycan property of having fucose residues, the database could include correlations between concentration 1 and glycan level 1, concentration 2 and glycan level 2, and so on. The correlative function can also be "tunable," e.g., it (or its output) can vary, e.g., in a linear or non-linear fashion, over a range of input values, according to a function. E.g., the correlative function can embody a function which relates X to Y, where X is a value for some element related to a production parameter and Y is a value for some element related to the glycan property (in some instances X is the input and Y is the output, in others Y is the input and X is the output). E.g., in the case where the production parameter is the presence of an additive, e.g., glucosamine, in the culture medium, X be the value for the concentration of glucosamine added to the culture medium. Y can be a value for the amount of fucose added to a protein made in a method which uses glucosamine at concentration X. In this instance, the correlative function relates a value (e.g., an input value) for concentration of glucosamine to a value (e.g., an output value) for the amount of fucosyl moieties on the glycoprotein. As the values for X increase, the values for Y will change according to the function which relates X and Y, and in the case of increasing glucosamine the output value will decrease. Thus, as the amount of glucosamine increases the correlative function indicates a lower amount of fucosylation. Such correlative functions are tunable in the sense that one can change the value of X and see the effect on Y. This allows tuning the production parameter to achieve a desired glycan property. The method also allows varying Y to see the effect on X. Functions relating X and Y can be determined, e.g., by empirical trial. E.g., in the case of glucosamine concentration and amount of fucosylation, a function can be derived by conducting a series of trials at different glucosamine concentrations, plotting glucosamine concentration against observed levels of fucosylation, and deriving an equation which describes the curve of the plotted values. In an instance, production parameter 1 is tunable for an input setting (or value) X1 and the output or setting (or value) for Y1 will vary with the setting (or value) of X1. Production parameter 2 is tunable for an input setting (or value) X2 and the output or setting (or value) for Y2 will vary with the setting (or value) of X2. In some instances the number of combinations of Y1 and Y2 is equal to the product of number of possibilities for Y1 and the number of possibilities for Y2. E.g., in a situation where there are 10 input values or settings for X1, 10 output values or settings for Y1, 10 input values or settings for X2, and 10 output values or settings for Y2, there are a total of a 100 combinations (of X 1X2 or Y1Y2) available. In other instances, some combinations of values or settings for X1 and X2, or some combination of values or settings for Y1 and Y2, are not compatible; either case results in a solution space, or total number of possibilities for the available combinations of Y1 and Y2 being less than the product of number of possibilities for Y1 and the number of possibilities for Y2 (or the analogous situation for X1X2). This constraint may be imposed by incompatibilities on combinations of X1 and X2, e.g., they may be concentrations of additives or combinations of additives and cells which cannot be combined for one reason or another. The constraint may also be imposed because a combination of Y 1 and Y2 are synthetically or structurally impossible or result in toxicity to the cell culture or to an unwanted property in a glycoprotein. A constraint, e.g., a physical or biological constraint, on solution space can be determined or elucidated, e.g., by empirical experimentation. The constraint of solution space (for X1X2 or Y1Y2) can be achieved in a database or system in a number of ways. E.g., the correlative function can be designed to produce a null output or a signal corresponding to an unavailable combination. This need not be absolute but could be expressed in degrees of undesirability. A system could be configured with a filter which identifies prohibited or unavailable combinations and labels them or removes them from output. The filter could be provided with specific unacceptable combinations or a rule-based algorithm for exclusion of unacceptable combinations. Nonlinear, constrained and pleiotropic correlations can be used in the methods, systems and databases described herein.

A correlative function, generally, is the degree to which one phenomenon or random variable (e.g., production parameter, glycoprotein function, etc.) is associated with or can be predicted from another. In statistics, correlation usually refers to the degree to which a linear predictive relationship exists between random variables, as measured by a correlation coefficient. Correlation may be positive (but never larger than 1), i.e., both variables increase or decrease together; negative or inverse (but never smaller than -1), i.e., one variable increases when the other decreases; or zero, i.e., a change in one variable does not affect the other.

Along with correlation functions (e.g., autocorrelations, cross-correlations, etc.), one or more stochastic processes, random variable theories or techniques, or probability theories may be used for identifying and selecting glycoprotein characteristics, production parameters, or other phenomenon or random variables and their relationships. For example, covariance functions, generalization functions, distributions functions, probability density functions and other types of mathematical representations may be implemented.

### Primary Glycoprotein Products

Methods described herein include identifying a primary glycoprotein product such as a naturally occurring or synthetically made product and producing a glycoprotein product having one or more preselected glycan properties. A primary glycoprotein product, as used herein, refers to a glycoprotein. The glycoprotein can serve as a model, starting or intermediate point for designing a glycoprotein product. It can provide or exhibit a desired glycoprotein property. Thus, in some instances, the preselected glycan properties can be the same or substantially similar to the preselected glycan properties of the primary glycoprotein product (e.g., to make a generic version of a primary glycoprotein product) or can be one or more glycan property that differs from the corresponding glycan property of the primary glycoprotein product (e.g., to make a second generation glycoprotein product). Exemplary primary glycoprotein products are provided in Table I below.

**Table I**

| **Protein Product** | **Reference Listed Drug** |
|---|---|
| interferon qamma-1b | Actimmune® |
| alteplase; tissue plasminogen activator | Activase®/Cathflo® |
| Recombinant antihemophilic factor | Advate |
| human albumin | Albutein® |
| Laronidase | Aldurazyme® |
| interferon alfa-N3, human leukocyte derived | Alferon N® |
| human antihemophilic factor | Alphanate® |
| virus-filtered human coaqulation factor IX | AlphaNine® SD |
| Alefacept; recombinant, dimeric fusion protein LFA3-Ig | Amevive® |
| Bivalirudin | Angiomax® |
| darbepoetin alfa | Aranesp™ |
| Bevacizumab | Avastin™ |
| interferon beta-1a; recombinant | Avonex® |
| coaqulation factor IX | BeneFix™ |
| Interferon beta-1b | Betaseron® |
| Tositumomab | BEXXAR® |
| antihemophilic factor | Bioclate™ |
| human growth hormone | BioTropin™ |
| botulinum toxin type A | BOTOX® |
| Alemtuzumab | Campath® |
| acritumomab; technetium-99 labeled | CEA-Scan® |
| alqlucerase; modified form of beta-glucocerebrosidase | Ceredase® |
| imiglucerase; recombinant form of beta-glucocerebrosidase | Cerezyme® |
| crotalidae polyvalent immune Fab, ovine | CroFab™ |
| digoxin immune fab [ovine] | DiqiFab™ |
| Rasburicase | Elitek® |
| Etanercept | ENBREL® |
| epoietin alfa | Epogen® |
| Cetuximab | Erbitux™ |
| algasidase beta | Fabrazyme® |
| Urofollitropin | Fertinex™ |
| follitropin beta | Follistim™ |
| Teriparatide | FORTEO® |
| human somatropin | GenoTropin® |
| Glucagon | GlucaGen® |
| follitropin alfa | Gonal-F® |
| antihemophilic factor | Helixate® |
| Antihemophilic Factor; Factor XIII | HEMOFIL |
| adefovir dipivoxil | Hepsera™ |
| Trastuzumab | Herceptin® |
| Insulin | Humalog® |
| antihemophilic factor/von Willebrand factor complex-human | Humate-P® |
| Somatotropin | Humatrope® |
| Adalimumab | HUMIRA™ |
| human insulin | Humulin® |
| recombinant human hyaluronidase | Hylenex™ |
| interferon alfacon-1 | Infergen® |
| eptifibatide | Integrilin™ |
| alpha-interferon | Intron A® |
| Palifermin | Kepivance |
| Anakinra | Kineret™ |
| antihemophilic factor | Kogenate®FS |
| insulin glargine | Lantus® |
| granulocyte macrophaqe colony-stimulating factor | Leukine®/Leukine® Liquid |
| lutropin alfa for injection | Luveris |
| OspA lipoprotein | LYMErix™ |
| Ranibizumab | LUCENTIS® |
| gemtuzumab ozogamicin | Mylotarg™ |
| Galsulfase | Naglazyme™ |
| Nesiritide | Natrecor® |
| Pegfilgrastim | Neulasta™ |
| Oprelvekin | Neumega® |
| Filgrastim | Neupogen® |
| Fanolesomab | NeutroSpec™ (formerly LeuTech®) |
| somatropin [rDNA] | Norditropin®/Norditropin Nordiflex® |
| Mitoxantrone | Novantrone® |
| insulin; zinc suspension; | Novolin L® |
| insulin; isophane suspension | Novolin N® |
| insulin, regular; | Novolin R® |
| Insulin | Novolin® |
| coagulation factor VIIa | NovoSeven® |
| Somatropin | Nutropin® |
| immunoglobulin intravenous | Octagam® |
| PEG-L-asparaqinase | Oncaspar® |
| abatacept, fully human soluable fusion protein | Orencia™ |
| muromomab-CD3 | Orthoclone OKT3® |
| high-molecular weight hyaluronan | Orthovisc® |
| human chorionic gonadotropin | Ovidrel® |
| live attenuated *Bacillus Calmette-Guerin* | Pacis® |
| peginterferon alfa-2a | Pegasys® |
| pegylated version of interferon alfa-2b | PEG-Intron™ |
| Abarelix (injectable suspension); gonadotropin-releasing hormone antagonist | Plenaxis™ |
| epoletin alfa | Procrit® |
| Aldesleukin | Proleukin, IL-2® |
| Somatrem | Protropin® |
| dornase alfa | Pulmozyme® |
| Efalizumab; selective, reversible T-cell blocker | RAPTIVA™ |
| combination of ribavirin and alpha interferon | Rebetron™ |
| Interferon beta 1a | Rebif® |
| antihemophilic factor | Recombinate® rAHF/ |
| antihemophilic factor | ReFacto® |
| Lepirudin | Refludan® |
| Infliximab | REMICADE® |
| Abciximab | ReoPro™ |
| Reteplase | Retavase™ |
| Rituxima | Rituxan™ |
| interferon alfa-2^{a} | Roferon-A® |
| Somatropin | Saizen® |
| synthetic porcine secretin | SecreFlo™ |
| Basiliximab | Simulect® |
| Eculizumab | SOLIRIS (R) |
| Pegvisomant | SOMAVERT® |
| Palivizumab; recombinantly produced, humanized mAb | Synagis™ |
| thyrotropin alfa | Thyrogen® |
| Tenecteplase | TNKase™ |
| Natalizumab | TYSABRI® |
| human immune globulin intravenous 5% and 10% solutions | Venoglobulin-S® |
| interferon alfa-n1, lymphoblastold | Wellferon® |
| drotrecogin alfa | Xigris™ |
| Omalizumab; recombinant DNA-derived humanized monoclonal antibody targeting immunoglobulin-E | Xolair® |
| Daclizumab | Zenapax® |
| ibritumomab tiuxetan | Zevalin™ |
| Somatotropin | Zorbtive™ (Serostim®) |

Methods described herein can include producing a target glycoprotein product that has the same amino acid sequence as the primary glycoprotein product. In other instances, the amino acid sequence of the target glycoprotein product can be differ, e.g., by up to 1, 2, 3, 4, 5, 10 or 20 amino acids, from the primary amino acid residues. The amino acid sequences of the primary glycoprotein products listed above are known.

### Methods of Determining Glycan Properties and Characteristics:

In some instances, the methods include selecting a production parameter or parameters to produce a preselected glycan property or properties. The glycan property can be a functional property or a glycan characteristic.

Methods for determining glycan characteristics are known. For example, the presence, absence or amount of a chemical unit or the presence, absence or amount of a component of a chemical unit may be determined as described by Geyer and Geyer (2006) Biochim Biophys. Acta 1764(12):1853-1869, or by LC, MS, LC/MS, NMR, exoglycosidase treatment, GC, or combinations of these methods. The heterogeneity or microheterogenity at a potential glycosylation site or across the entire protein can be determined, e.g., using the methods described by Larsen et al. (2005) Mol. Cell. Proteomics (2005) 4(2):107-119 or Forno et al. (2004 Eur. J. Biochem. (2004) 271(5): 907-919, or LC, MS, LC/MS, GC, PAGE, enzymatic treatment, or combinations of these methods.

In some instances, the core structure of a branched or unbranched glycan is determined, e.g., as described by Geyer and Geyer (2006) *supra,* LC, MS, LC/MS, lectin staining, GC, PAGE, enzymatic cleavage or addition, ELISA, NMR, monosaccharide analysis, or combinations of these methods on the intact glycoprotein, glycopeptides, or released glycan. Exemplary methods that can be used to determine the presence, absence or amount of a glycan structure and the relative position of a chemical unit within a glycan are described by Geyer and Geyer (2006) *supra,* or can include LC, MS, LC/MS, lectin staining, chromatographic methods, enzymatic cleavage, ELISA quantitation, monosaccharide analysis NMR, or combinations of methods therein on the glycoprotein, glycopeptides, or released glycan.

The relationship between chemical units (e.g., linkages between chemical units, isomers and branch points) can be determined, e.g., as described by Geyer and Geyer in Biochim Biophys. Acta (2006) *supra,* or by LC, MS, LC/MS, lectin staining, monosaccharide analysis, chromatographic methods, exoglycosidase treatment, NMR, or combinations of methods therein on the glycoprotein, glycopeptides or released glycan.

In some instances, information about a glycan structure or structures, e.g., obtained by a method described herein, can be integrated to describe the glycan characteristics of a complex glycoprotein product. For example, information obtained, e.g., by various methods described herein, can be used in a step by step manner to reduce the initial possibilities of glycan characteristics in a primary glycan product and/or a target glycan product. In one instance, the data obtained regarding various glycan characteristics can be integrated using the methods described in U.S. Patent Publication No: 20050065738.

### Production Parameters:

Methods described herein include determining and/or selecting a production parameter or parameters for a glycoprotein preparation such that a preselected glycan property or properties can be obtained upon production of a glycoprotein preparation. By using information regarding the effects of various production parameters on glycosylation, production parameters can be selected prior to the production of a glycoprotein preparation that positively correlate with the desired glycan properties. A production parameter as used herein is a parameter or element in a production process. Production parameters that can be selected include, e.g., the cell or cell line used to produce the glycoprotein preparation, the culture medium, culture process or bioreactor variables (e.g., batch, fed-batch, or perfusion), purification process and formulation of a glycoprotein preparation.

Primary production parameters include: 1) the types of host; 2) genetics of the host; 3) media type; 4) fermentation platform; 5) purification steps; and 6) formulation. Secondary production parameter, as used herein, is a production parameter that is adjustable or variable within each of the primary production parameters. Examples include: selection of host subclones based on desired glycan properties; regulation of host gene levels constitutive or inducible; introduction of novel genes or promoter elements; media additives (e.g. partial list on Table IV); physiochemical growth properties (e.g. partial list on Table V); growth vessel type (e.g. bioreactor type, T flask); cell density; cell cycle; enrichment of product with a desired glycan type (e.g. by lectin or antibody-mediated enrichment, ionexchange chromatography, CE, or similar method); or similar secondary production parameters clear to someone skilled in the art.

### Cells & Cell Lines

Methods described herein can include determining a cell or cell line to provide a glycan property that is the same or substantially the same as a glycan property of a primary glycoprotein preparation or that differs from a glycan property of a primary glycoprotein product. The selected cell can be eukaryotic or prokaryotic, as long as the cell provides or has added to it the enzymes to activate and attach saccharides present in the cell or saccharides present in the cell culture medium or fed to the cells. Examples of eukaryotic cells include yeast, insect, fungi, plant and animal cells, especially mammalian cells. Suitable mammalian cells include any normal mortal or normal or abnormal immortal animal or human cell, including: monkey kidney CV 1 line transformed by SV40 (COS-7, ATCC CRL 1651); human embryonic kidney line (293) (Graham et al., J. Gen. Virol. 36:59 (1977)); baby hamster kidney cells (BHK, ATCC CCL 10); Chinese Hamster Ovary (CHO), e.g., DG44, DUKX-V 11, GS-CHO (ATCC CCL 61, CRL 9096, CRL 1793 and CRL 9618); mouse sertoli cells (TM4, Mather, Biol. Reprod. 23:243 251 (1980)); monkey kidney cells (CV1 ATCC CCL 70); African green monkey kidney cells (VERO-76, ATCC CRL 1587); human cervical carcinoma cells (HeLa, ATCC CCL 2); buffalo rat liver cells (BRL 3A, ATCC CRL 1442); human lung cells (W138, ATCC CCL 75); human liver cells (Hep G2, HB 8065); mouse melanoma cells (NSO); mouse mammary tumor (MMT 060562, ATCC CCL51), TRI cells (Mather, et al., Annals N.Y. Acad. Sci. 383:44 46 (1982)); canine kidney cells (MDCK) (ATCC CCL 34 and CRL 6253), HEK 293 (ATCC CRL 1573), WI-38 cells (ATCC CCL 75) (ATCC: American Type Culture Collection, Rockville, Md.), MCF-7 cells, MDA-MB-438 cells, U87 cells, A127 cells, HL60 cells, A549 cells, SP10 cells, DOX cells, SHSY5Y cells, Jurkat cells, BCP-1 cells, GH3 cells, 9L cells, MC3T3 cells, C3H-10T1/2 cells, NIH-3T3 cells and C6/36 cells. The use of mammalian tissue cell culture to express polypeptides is discussed generally in Winnacker, FROM GENES TO CLONES (VCH Publishers, N.Y., N.Y., 1987).

Exemplary plant cells include, for example, *Arabidopsis thaliana,* rape seed, corn, wheat, rice, tobacco etc.) (Staub, et al. 2000 Nature Biotechnology 1(3): 333-338 and McGarvey, P. B., et al. 1995 Bio-Technology 13(13): 1484-1487; Bardor, M., et al. 1999 Trends in Plant Science 4(9): 376-380). Exemplary insect cells (for example, *Spodoptera frugiperda Sf9,* Sf21, *Trichoplusia ni,* etc. Exemplary bacteria cells include *Escherichia coli*. Various yeasts and fungi such as *Pichiapastoris, Pichia methanolica, Hansenula polymorpha,* and *Saccharomyces cerevisiae* can also be selected.

A cell can be selected for production of a glycoprotein based, e.g., upon attributes of the cell itself which produce or show a preference for production of the desired glycan characteristic or characteristics. Attributes of the cell that may affect glycosylation include the type of cell, cell state, the cell cycle, the passage number, and the metabolic stress level of the cell.

In other instances, a glycoprotein can be produced in a genetically engineered cell, e.g., a genetically engineered animal, yeast, fungi, plants, or other eukaryotic cell expression system. For example, a cell can be a genetically engineered cell which expresses or over expresses a component, e.g., a protein and/or sugar or sugar precursor, which produces a desired glycan characteristic or characteristics. A cell can also be genetically engineered such that the activity of a component, e.g., a protein and/or sugar or sugar precursor, which produces a desired glycan characteristic or characteristics, is increased. The cell can also be genetically engineered to decrease or reduce production of various chemical units, components of chemical units or glycan structures. For example, the cell can be genetically engineered to produce a nucleic acid antagonist such as antisense or RNAi which results in decreased expression of component involved with the synthesis of a particular glycan characteristic, e.g., an enzyme and/or sugar or sugar precursor involved in the production of a glycan characteristic or characteristics. The cell can also be genetically engineered to knock out one or more components, e.g., an enzyme and/or sugar or sugar precursor, involved with the synthesis of a particular glycan characteristic, or to produce a less active or inactive mutant of a component, e.g., an enzyme and/or sugar or sugar precursor, involved with synthesis of a particular glycan characteristic or characteristics. The copy number, site of integration and transcription variables can affect the glycan characteristics of a glycoprotein produced by the cell.

Components of a cell that result in a desired glycan characteristic or characteristic can include enzymes involved with the addition or removal of a chemical unit, a component of a chemical unit, or production of a desired glycan structure. In some instances, the cell can be genetically engineered to expresses, overexpress or otherwise increase the activity of one or more enzymes involved in glycosylation. Other instances include a cell genetically engineered to reduce, eliminate or otherwise alter the activity of one or more enzymes involved in glycosylation. Exemplary enzymes include enzymes that cleave polysaccharides such as degrading enzymes, enzymes that add monoshaccharides to a glycan structure, enzymes that remove a component of a monosaccharide, enzymes that add a component to a monosaccharide and enzymes that convert a chemical unit into a different chemical unit, e.g., convert galactose to a glucose, etc.

Examples of degrading enzymes include a galactosidase (e.g., alpha-galactosidase and beta-galactosidase), a sialidase (e.g., an alpha 2→3 sialidase and an alpha 2→6 sialidase), a fucosidase (e.g., an alpha 1→2 fucosidase, a alpha 1→3 fucosidase, an alpha 1→4 fucosidase and an alpha 1→6 fucosidase. beta -N-Acetylhexosaminidase from Jack Bean cleave non-reducing terminal beta 1→2,3,4,6 linked N-acetylglucosamine, and N-acetylgalactosamine from oligosaccharides whereas alpha-N-Acetylgalactosaminidase (Chicken liver) cleaves terminal alpha 1→3 linked N-acetylgalactosamine from glycoproteins. Other enzymes such as aspartyl-N-acetylglucosaminidase cleave at a beta linkage after a GlcNAc in the core sequence of N-linked oligosaccharides.

Examples of enzymes which add a monosaccharide to a glycan structure include glycosyltransferases such as a sialyltransferase (e.g., alpha 2→3 sialyltransferase or alpha 2→6 sialyltransferase), a fucosyltransferase (e.g., alpha 1→2 fucosyltransferse, alpha 1→3 fucosyltransferase, alpha 1→4 fucosyltransferase or alpha 1→6 fucosyltransferase), a galactosyltransferase (e.g., alpha 1→3 galactosyltransferase, beta 1→4 galactosyltransferase or beta 1→3 galactosyltransferase), a N-acetylglucosaminyltransferase (e.g., N-acetylglucosaminyltransferase I, II or III), and a mannosyltransferase.

Examples of enzymes which add, transfer or remove a component of a monosaccharide include: glucoseamine N-acetyl transferase, N-acetylneuraminate 7-0 (or 9-0) acetyl transferase, galactose-1-phosphate uridyltransferase, N-acetylneuraminate 9-phosphate phosphotase, N-acetylglucoasamine deacetylase, L-fucose kinase, galactokinase 1, galactose-1-phosphate uridylyltransferase, glucokinase 1, GDP-mannose 4,6 dehydratase, GDP mannose pyrophosphorylase, N-acetylglucosamine sulfotransferase, galactosyl sulfotransferase, glucosamine-phosphate N-acetyl transferase, hexokinase, N-acetylglucosamine kinase, phosphoglucomutase, N-acetylneuraminic acid phosphate synthetase, UDP-N-GlcNAc-pyrophosphorylase, UDP-glucuronate dehydrogenase, and UDP-glucose pyrophosphorylase.

Other exemplary enzymes that can be affected in a genetically engineered cell include N-acetylglucosamine-6-phosphate 2-epimerase, CMP-Neu5Ac hydroxylase, CMP-Neu5Ac synthetase, cyclic sialic acid hydrolase, fucose-1-phosphate guanyltransferase, UDP-galactose-4-epimerase, galactose mutaratose, mannosyltransferase, UDP-N-acetylglucosamine 2-epimerase, glucose phosphate isomerase, GDP-mannosyl transferase, mannose phosphate isomerase, N-acetylneuraminate pyruvate lyase, sialic acid cyclase, UDP-glucuronate decarboxylase, CMP-sialic acid transporter, GDP-fucosyl transporter and UDP galactosyl transporter.

The sequences encoding such enzymes are known.

A selected cell for production of a glycoprotein can be a genetically engineered cell that has decreased the expression and/or activity of one or more proteins involved in the glycosylation. For example, the cell can be genetically engineered to knock out one or more proteins involved with the synthesis of a particular glycan characteristic or to produce a less active or inactive mutant of a protein. A cell can also be genetically engineered to produce a nucleic acid antagonist to decrease expression of one or more proteins involved with the synthesis of a particular glycan characteristic.

### Genetically Engineered Knock Out Cells

In some instances, a cell can be selected which has been genetically engineered for permanent or regulated inactivation of a gene encoding a protein involved with the synthesis of a particular glycan. For example, genes encoding an enzyme such as the enzymes described herein can be inactivated. Permanent or regulated inactivation of gene expression can be achieved by targeting to a gene locus with a transfected plasmid DNA construct or a synthetic oligonucleotide. The plasmid construct or oligonucleotide can be designed to several forms. These include the following: 1) insertion of selectable marker genes or other sequences within an exon of the gene being inactivated; 2) insertion of exogenous sequences in regulatory regions of non-coding sequence; 3) deletion or replacement of regulatory and/or coding sequences; and, 4) alteration of a protein coding sequence by site specific mutagenesis.

In the case of insertion of a selectable marker gene into coding sequence, it is possible to create an in-frame fusion of an endogenous exon of the gene with the exon engineered to contain, for example, a selectable marker gene. In this way following successful targeting, the endogenous gene expresses a fusion mRNA (nucleic acid sequence plus selectable marker sequence). Moreover, the fusion mRNA would be unable to produce a functional translation product.

In the case of insertion of DNA sequences into regulatory regions, the transcription of a gene can be silenced by disrupting the endogenous promoter region or any other regions in the 5' untranslated region (5' UTR) that is needed for transcription. Such regions include, for example, translational control regions and splice donors of introns. Secondly, a new regulatory sequence can be inserted upstream of the gene that would render the gene subject to the control of extracellular factors. It would thus be possible to down-regulate or extinguish gene expression as desired for glycoprotein production. Moreover, a sequence which includes a selectable marker and a promoter can be used to disrupt expression of the endogenous sequence. Finally, all or part of the endogenous gene could be deleted by appropriate design of targeting substrates.

### Nucleic Acid Antagonists

In certain implementations, nucleic acid antagonists are used to decrease expression of a target protein, e.g., a protein involved with the synthesis of a glycan characteristic, e.g., an enzyme such as those discussed above. In one instance, the nucleic acid antagonist is an siRNA that targets mRNA encoding the target protein. Other types of antagonistic nucleic acids can also be used, e.g., a nucleic acid aptamer, a dsRNA, a ribozyme, a triple-helix former, or an antisense nucleic acid.

siRNAs are small double stranded RNAs (dsRNAs) that optionally include overhangs. For example, the duplex region of an siRNA is about 18 to 25 nucleotides in length, e.g., about 19, 20, 21, 22, 23, or 24 nucleotides in length. Typically the siRNA sequences are exactly complementary to the target mRNA. dsRNAs and siRNAs in particular can be used to silence gene expression in mammalian cells (e.g., human cells). See, e.g., Clemens, J. C. et al. (2000) Proc. Natl. Sci. USA 97, 6499-6503; Billy, E. et al. (2001) Proc. Natl. Sci. USA 98, 14428-14433; Elbashir et al. (2001) Nature. 411(6836):494-8; Yang, D. et al. (2002) Proc. Natl. Acad. Sci. USA 99, 9942-9947, US 2003-0166282, 2003-0143204, 2004-0038278, and 2003-0224432.

Anti-sense agents can include, for example, from about 8 to about 80 nucleobases (i.e. from about 8 to about 80 nucleotides), e.g., about 8 to about 50 nucleobases, or about 12 to about 30 nucleobases. Anti-sense compounds include ribozymes, external guide sequence (EGS) oligonucleotides (oligozymes), and other short catalytic RNAs or catalytic oligonucleotides which hybridize to the target nucleic acid and modulate its expression. Anti-sense compounds can include a stretch of at least eight consecutive nucleobases that are complementary to a sequence in the target gene. An oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired.

Hybridization of antisense oligonucleotides with mRNA can interfere with one or more of the normal functions of mRNA. The functions of mRNA to be interfered with include all vital functions such as, for example, translocation of the RNA to the site of protein translation, translation of protein from the RNA, splicing of the RNA to yield one or more mRNA species, and catalytic activity which may be engaged in by the RNA. Binding of specific protein(s) to the RNA may also be interfered with by antisense oligonucleotide hybridization to the RNA.

Exemplary antisense compounds include DNA or RNA sequences that specifically hybridize to the target nucleic acid. The complementary region can extend for between about 8 to about 80 nucleobases. The compounds can include one or more modified nucleobases. Modified nucleobases may include, e.g., 5-substituted pyrimidines such as 5-iodouracil, 5-iodocytosine, and C5-propynyl pyrimidines such as C5-propynylcytosine and C5-propynyluracil. Other suitable modified nucleobases include N4 --(C1 - C12)alkylaminocytosines and N4,N4 --(C1 -C12)dialkylaminocytosines. Modified nucleobases may also include 7-substituted-8-aza-7-deazapurines and 7-substituted-7-deazapurines such as, for example, 7-iodo-7-deazapurines, 7-cyano-7-deazapurines, 7-aminocarbonyl-7-deazapurines. Examples of these include 6-amino-7-iodo-7-deazapurines, 6-amino-7-cyano-7-deazapurines, 6-amino-7-aminocarbonyl-7-deazapurines, 2-amino-6-hydroxy-7-iodo-7-deazapurines, 2-amino-6-hydroxy-7-cyano-7-deazapurines, and 2-amino-6-hydroxy-7-aminocarbonyl-7-deazapurines. Furthermore, N6 --(C1-C12)alkylaminopurines and N6,N6 --(C1 -C12)dialkylaminopurines, including N6-methylaminoadenine and N6,N6 -dimethylaminoadenine, are also suitable modified nucleobases. Similarly, other 6-substituted purines including, for example, 6-thioguanine may constitute appropriate modified nucleobases. Other suitable nucleobases include 2-thiouracil, 8-bromoadenine, 8-bromoguanine, 2-fluoroadenine, and 2-fluoroguanine. Derivatives of any of the aforementioned modified nucleobases are also appropriate. Substituents of any of the preceding compounds may include C1-C30 alkyl, C2 -C30 alkenyl, C2 -C30 alkynyl, aryl, aralkyl, heteroaryl, halo, amino, amido, nitro, thio, sulfonyl, carboxyl, alkoxy, alkylcarbonyl, alkoxycarbonyl, and the like.

Descriptions of other types of nucleic acid agents are also available. See, e.g., US 4,987,071; US 5,116,742; US 5,093,246; Woolf et al. (1992) Proc Natl Acad Sci USA; Antisense RNA and DNA, D. A. Melton, Ed., Cold Spring Harbor Laboratory, Cold Spring Harbor, N.Y. (1988); 89:7305-9; Haselhoff and Gerlach (1988) Nature 334:585-59; Helene, C. (1991) Anticancer Drug Des. 6:569-84; Helene (1992) Ann. N.Y. Acad. Sci. 660:27-36; and Maher, L.J. (1992) Bioassays 14:807-15.

### Cells Genetically Engineered to Express a Component Involved in Glycan Synthesis

When cells are to be genetically modified for the purposes of expressing or overexpressing a component, the cells may be modified by conventional genetic engineering methods or by gene activation.

According to conventional methods, a DNA molecule that contains cDNA or genomic DNA sequence encoding desired protein may be contained within an expression construct and transfected into primary, secondary, or immortalized cells by standard methods including, but not limited to, liposome-, polybrene-, or DEAE dextran-mediated transfection, electroporation, calcium phosphate precipitation, microinjection, or velocity driven microprojectiles (see, e.g., U.S. Patent No. 6,048,729).

Alternatively, one can use a system that delivers the genetic information by viral vector. Viruses known to be useful for gene transfer include adenoviruses, adeno associated virus, herpes virus, mumps virus, pollovirus, retroviruses, Sindbis virus, and vaccinia virus such as canary pox virus.

Alternatively, the cells may be modified using a gene activation approach, for example, as described in U.S. Patent No. 5,641,670; U.S. Patent No. 5,733,761; U.S. Patent No. 5,968,502; U.S. Patent No. 6,200,778; U.S. Patent No. 6,214,622; U.S. Patent No. 6,063,630; U.S. Patent No. 6,187,305; U.S. Patent No. 6,270,989; and U.S. Patent No. 6,242,218.

Accordingly, the term "genetically engineered," as used herein in reference to cells, is meant to encompass cells that express a particular gene product following introduction of a DNA molecule encoding the gene product and/or including regulatory elements that control expression of a coding sequence for the gene product. The DNA molecule may be introduced by gene targeting or homologous recombination, i.e., introduction of the DNA molecule at a particular genomic site.

Methods of transfecting cells, and reagents such as promoters, markers, signal sequences which can be used for recombinant expression are known.

In some instances, the promoter and/or expression system can be selected as, e.g., a secondary production parameter. For example, the promoter can be selected, e.g., based upon the host cell being used.

### Culture Media and Processing:

The methods described herein can include determining and/or selecting media components or culture conditions which result in the production of a desired glycan property or properties. Culture parameters that can be determined include media components, pH, feeding conditions, osmolarity, carbon dioxide levels, agitation rate, temperature, cell density, seeding density, timing and sparge rate.

Changes in production parameters such the speed of agitation of a cell culture, the temperature at which cells are cultures, the components in the culture medium, the times at which cultures are started and stopped, variation in the timing of nutrient supply can result in variation of a glycan properties of the produced glycoprotein product. Thus, methods described herein can include one or more of: increasing or decreasing the speed at which cells are agitated, increasing or decreasing the temperature at which cells are cultures, adding or removing media components, and altering the times at which cultures are started and/or stopped.

Sequentially selecting a production parameters or a combination thereof, as used herein, means a first parameter (or combination) is selected, and then a second parameter (or combination) is selected, e.g., based on a constraint imposed by the choice of the first production parameter.

### Media

The methods described herein can include determining and/or selecting a media component and/or the concentration of a media component that has a positive correlation to a desired glycan property or properties. A media component can be added in or administered over the course of glycoprotein production or when there is a change in media, depending on culture conditions. Media components include components added directly to culture as well as components that are a byproduct of cell culture.

Media components include, e.g., buffer, amino acid content, vitamin content, salt content, mineral content, serum content, carbon source content, lipid content, nucleic acid content, hormone content, trace element content, ammonia content, co-factor content, indicator content, small molecule content, hydrolysate content and enzyme modulator content. Table IV provides examples of various media components that can be selected.

| **Table IV** | |
|---|---|
| amino acids | sugar precursors |
| Vitamins | Indicators |
| Carbon source (natural and unnatural) | Nucleosides or nucleotides |
| Salts | butyrate or organics |
| Sugars | DMSO |
| Sera | Animal derived products |
| Plant derived hydrolysates | Gene inducers |
| sodium pyruvate | Non natural sugars |
| Surfactants | Regulators of intracellular pH |
| Ammonia | Betaine or osmoprotectant |
| Lipids | Trace elements |
| Hormones or growth factors | minerals |
| Buffers | Non natural amino acids |
| Non natural amino acids | Non natural vitamins |

Exemplary buffers include Tris, Tricine, HEPES, MOPS, PIPES, TAPS, bicine, BES, TES, cacodylate, MES, acetate, MKP, ADA, ACES, glycinamide and acetamidoglycine.

The media can be serum free or can include animal derived products such as, e.g., fetal bovine serum (FBS), fetal calf serum (FCS), horse serum (HS), human serum, animal derived serum substitutes (e.g., Ultroser G, SF and HY; non-fat dry milk; Bovine EX-CYTE), fetuin, bovine serum albumin (BSA), serum albumin, and transferrin. When serum free media is selected lipids such as, e.g., palmitic acid and/or steric acid, can be included.

Lipids components include oils, saturated fatty acids, unsaturated fatty acids, glycerides, steroids, phospholipids, sphingolipids and lipoproteins.

Exemplary amino acid that can be included or eliminated from the media include alanine, arginine, asparagine, aspartic acid, cysteine, glutamic acid, glutamine, glycine, histidine, proline, isoleucine, leucine, lysine, methionine, phenylalanine, proline, serine, threonine, tryptophan, tyrosine and valine.

Examples of vitamins that can be present in the media or eliminated from the media include vitamin A (retinoid), vitamin B1 (thiamine), vitamin B2 (riboflavin), vitamin B3 (niacin), vitamin B5 (pantothenic acid), vitamin B6 (pyroxidone), vitamin B7 (biotin),vitamin B9 (folic acid), vitamin B12 (cyanocobalamin), vitamin C (ascorbic acid), vitamin D, vitamin E, and vitamin K.

Minerals that can be present in the media or eliminated from the media include bismuth, boron, calcium, chlorine, chromium, cobalt, copper, fluorine, iodine, iron, magnesium, manganese, molybdenum, nickel, phosphorus, potassium, rubidium, selenium, silicon, sodium, strontium, sulfur, tellurium, titanium, tungsten, vanadium, and zinc. Exemplary salts and minerals include CaCl₂ (anhydrous), CuSO₄ 5H₂O, Fe(NO₃) •9H₂O, KCl, KNO₃, KH₂PO₄, MgSO₄ (anhydrous), NaCl, NaH₂PO₄H₂O, NaHCO₃, Na₂SE₃ (anhydrous), ZnSO₄•7H₂O; linoleic acid, lipoic acid, D-glucose, hypoxanthine 2Na, phenol red, putrescine 2HCl, sodium pyruvate, thymidine, pyruvic acid, sodium succinate, succinic acid, succinic acid • Na • hexahydrate, glutathione (reduced), para-aminobenzoic acid (PABA), methyl linoleate, bacto peptone G, adenosine, cytidine, guanosine, 2'-deoxyadenosine HCl, 2'-deoxycytidine HCl, 2'-deoxyguanosine and uridine. When the desired glycan characteristic is decreased fucosylation, the production parameters can include culturing a cell, e.g., CHO cell, e.g., dhfr deficient CHO cell, in the presence of manganese, e.g., manganese present at a concentration of about 0.1 µM to 50 µM. Decreased fucosylation can also be obtained, e.g., by culturing a cell (e.g., a CHO cell, e.g., a dhfr deficient CHO cell) at an osmolality of about 350 to 500 mOsm. Osmolality can be adjusted by adding salt to the media or having salt be produced as a byproduct as evaporation occurs during production.

Hormones include, for example, somatostatin, growth hormone-releasing factor (GRF), insulin, prolactin, human growth hormone (hGH), somatotropin, estradiol, and progesterone. Growth factors include, for example, bone morphogenic protein (BMP), epidermal growth factor (EGF), basic fibroblast growth factor (bFGF), nerve growth factor (NGF), bone derived growth factor (BDGF), transforming growth factor- beta1 (TGF-betal), [Growth factors from US 6,838,284 B2], hemin and NAD.

Examples of surfactants that can be present or eliminated from the media include Tween-80 and pluronic F-68.

Small molecules can include, e.g., butyrate, ammonia, non natural sugars, non natural amino acids, chloroquine, and betaine.

In some instances, ammonia content can be selected as a production parameter to produce a desired glycan characteristic or characteristics. For example, ammonia can be present in the media in a range from 0.001 to 50 mM. Ammonia can be directly added to the culture and/or can be produced as a by product of glutamine or glucosamine. When the desired glycan characteristic is one or more of an increased number of high mannose structures, decreased fucosylation and decreased galactosylation, the production parameters selected can include culturing a cell (e.g., a CHO cell, e.g., a dhfr deficient CHO cell) in the presence of ammonia, e.g., ammonia present at a concentration of about 0.01 to 50 mM. For example, if the desired glycan characteristic includes decreased galactosylation, production parameters selected can include culturing a cell (e.g., a CHO cell, e.g., a dhfr deficient CHO cell) in serum containing media and in the presence of ammonia, e.g., ammonia present at a concentration of about 0.01 to 50 mM.

Another production parameter is butyrate content. The presence of butyrate in culture media can result in increased galactose levels in the resulting glycoprotein preparation. Butyrate provides increased sialic acid content in the resulting glycoprotein preparation. Therefore, when increased galactosylation and/or sialylation is desired, the cell used to produce the glycoprotein (e.g., a CHO cell, e.g., a dhfr deficient CHO cell) can be cultured in the presence of butyrate. In some instances, butyrate can be present at a concentration of about 0.001 to 10 mM, e.g., about 2 mM to 10 mM. For example, if the desired glycan characteristic includes increased sialylation, production parameters selected can include culturing a cell (e.g., a CHO cell, e.g., a dhfr deficient CHO cell) in serum containing media and in the presence of butyrate, e.g., butyrate present at a concentration of about 2.0 to 10 mM. Such methods can further include selecting one or more of adherent culture conditions and culture in a T flask.

In some instances, a component such as an enzyme, sugar and/or sugar precursors can be added to media or batch fed to cells to affect glycosynthesis. For example, enzymes and substrates such as sugar precursors can be added to the media or batch fed to cells to produce a desired glycan characteristic or characteristics. These methods can make use of monosaccharide substrates that are taken up by a cell, converted to "activated" monosaccharide substrates *in vivo* and incorporated into the expressed protein by the cell. The methods are amenable to any cell which can be manipulated to produce a desired glycoprotein. The cell can use, e.g., endogenous biochemical processing pathways or can be genetically engineered to convert, or process, the exogenously added monosaccharide into an activated form that serves as a substrate for conjugation to a target glycoprotein *in vivo* or *in vitro.*

Monosaccharides added to a polysaccharide chain can be incorporated in activated form. Activated monosaccharides, which can be added, include UDP-galactose, UDP-glucose, UDP-N-acetylglucosamine, UDP-N-acetylgactosamine, UDP-xylose, GDP-mannose, GDP-fucose, CMP-N-acetylneuraminic acid and CMP-N-acetylglycolylneuraminic acid. Other monosaccharide precursors that can be added to media or batch fed to cells include: N-acetylglucosamine, glucosamine, glucose, galactose, N-acetylgalactosamine, fructose, fucose, glucose-6-phosphate, mannose-6- phosphate, mannose-1-phosphate, fructose-6-phosphate, glucosamine-6-phosphate, N-acetylglucosamine-6-phosphate, N-acetylmannosamine, N-acetylneuraminic acid-6-phosphate, fucose-1-phosphate, ATP, GTP, GDP, GMP, CTP, CDP, CMP, UTP, UDP, UMP, uridine, adenosine, guanosine, cytodine, lactose, maltose, sucrose, fructose 1,6 biphosphate, 2 phosphoenol pyruvate, 2-oxaloacetate and pyruvate.

Activated forms of monosaccharides can be generated by methods known in the art. For example, galactose can be activated to UDP-galactose by several ways including: direct phosphorylation at the 1-position to give Gal-1-P, which can react with UTP to give UDP-galactose; Gal-1-P can be converted to UDP-galactose via uridyl transferase exchange reaction with UDP-glucose that displaces Glc-1-P. UDP-glucose can be derived from glucose by converting glucose to Glc-6-P by hexokinase and then either to Fru-6-P by phosphoglucose isomerase or to Glc-1-P by phosphoglucomutase. Reaction of Glc-1-P with UTP forms UDP-glucose. GDP-fucose can be derived from GDP-Man by reduction with CH₂OH at the C-6 position of mannose to a CH₃. This can be done by the sequential action of two enzymes. First, the C-4 mannose of GDP-Man is oxidized to a ketone, GDP-4-dehydro-6-deoxy-mannose, by GDP-Man 4,6-dehydratase along with reduction of NADP to NADPH. The GDP-4-keto-6-deoxymannose is the epimerized at C-3 and C-5 to form GDP-4-keto-6-deoxyglucose and then reduced with NADPH at C-4 to form GDP-fucose. Methods of obtaining other activated monosaccharide forms can be found in, e.g., Varki, A et al., eds., Essentials of Glycobiology, Cold Spring Harbor Press, Cold Spring Harbor, NY (1999).

An activated monosaccharide can be incorporated into a polysaccharide chain using the appropriate glycosyltransferase. For example, to incorporate a sialic acid, CMP-sialic acid onto a polysaccharide chain, a sialyltransferase, e.g., alpha 2→3 sialyltransferase or alpha 2→6 sialyltransferase, can be used. To incorporate a fucose, a fucosyltransferase, e.g., alpha 1→2 fucosyltransferse, alpha 1→3 fucosyltransferase, alpha 1→4 fucosyltransferase or alpha 1→6 fucosyltransferase, can be used. Glycosyltransferases for incorporating galactose and GlcNAc include a galactosyltransferase (e.g., alpha 1→3 galactosyltransferase, beta 1→4 galactosyltransferase or beta 1→3 galactosyltransferase) and a N-acetylglucosaminyltransferase (e.g., N-acetylglucosaminyltransferase I, II or III), respectively. Glycosyltransferases for incorporating other monsaccharides are known. The glycosyltransferase can be added to the media or batch fed to the cell or the cell can use endogenous processing pathways or be genetically engineered to convert or process the exogenously added monosaccharide.

Other examples of enzymes that can be added to the media or batch fed to the cell are described herein.

Some aspects include having glucosamine present in the media. Glucosamine can be added to the media or batch fed to the cell or the appropriate enzymes and/or substrates can be added to the media or batch fed to cells such that glucosamine is produced. For example, one or more of N-acetylglucosamine, N-acetylglucosamine 6-phosphate, N-acetylmannosamine or fructose can be added to the media or batch fed to the cell for production of glucosamine. Cells cultured in the presence of glucosamine can provide decreased levels of fucosylation and/or galactosylation. Thus, in some instances, when reduced fucosylation and/or galactosylation is desired, a cell (e.g., a CHO cell, e.g., a dhfr deficient CHO cell) can be cultured, e.g., in serum containing media, in the presence of glucosamine. The presence of glucosamine in cell culture can also increase the amount of high mannose structures and hybrid structures in a glycoprotein preparation. Thus, in some instances, when increased levels of high mannose or hybrid glycan structures are desired, a cell (e.g., a CHO cell, e.g., a dhfr deficient CHO cell) can be cultured in the presence of glucosamine. Glucosamine can be present, e.g., at a concentration of about 0.001 to 40 mM.

The methods can further include having uridine added to the media or batch fed to a cell, e.g., to reduce the level of high mannose structures associated with a protein produced by the cell. The addition of cytidine, UTP, OMP and/or aspartate to media or batch fed to cells can also result in the production of uridine during culture. Preferably, uridine is present at a concentration of about 0.001 to 10 mM.

Other aspects include selecting a media component or components that do not significantly affect a glycosylation characteristic or characteristics. For example, the presence of glucosamine and uridine in culture does not significantly alter galactosylation, fucosylation, high mannose production, hybrid production or sialylation of glycoproteins produced by a cell (e.g., a CHO cell, e.g., a dhfr deficient CHO cell) cultured in the presence of this combination. In addition, the presence of mannose in culture does not significantly alter galactosylation, fucosylation, high mannose production, hybrid production or sialylation of glycoproteins produced by a cell (e.g., a CHO cell, e.g., a dhfr deficient CHO cell) cultured in the presence of mannose. Thus, the methods described herein can include selecting a media component such as mannose and/or the combination of glucosamine and uridine such that the glycan characteristic or characteristic is not significantly altered by this component (or components) of the media.

When the presence of mannose is a selected production parameter, mannose can be added to the media, batch fed to the cells or can be produced by a cell exposed to the appropriate substrates such as fructose or mannan. Preferably, mannose is present at a concentration of about 0.001 to 50 mM.

Various production parameters including media components and culture conditions (Column A) and the effect on a glycan characteristic (Row A) are described below in Table II.

**Table II:**

| **A** | **Galactosylation** | **Fucosylation** | **High Mannose** | **Hybrid** | **Sialylation** |
|---|---|---|---|---|---|
| **Mannose** | | | | | |
| **Glucosamine** | Decreased | Decreased | Increased | Increased | |
| **ManNAc** | | | | | |
| **Butyrate** | Increased | | | | |
| **450 mOsm** | | Decreased | | | |
| **Ammonia** | Decreased | Decreased | Increased | | |
| **32C** | | | | | |
| **15% CO2** | | Decreased | | | |
| **Manganese** | | Decreased | | | |
| **Glucosamine with Uridine** | | | | | |
| **Uridine** | | | Decreased | | |

### Physiochemical Parameters

Methods described herein can include selecting culture conditions that are correlated with a desired glycan property or properties. Such conditions can include temperature, pH, osmolality, shear force or agitation rate, oxidation, spurge rate, growth vessel, tangential flow, DO, CO₂, nitrogen, fed batch, redox, cell density and feed strategy. Examples of physiochemical parameters that can be selected are provided in Table V.

| **Table V:** | |
|---|---|
| Temperature | DO |
| pH | CO₂ |
| osmolality | Nitrogen |
| shear force, or agitation rate | Fed batch |
| oxidation | Redox |
| Spurge rate | Cell density |
| Growth vessel | Perfusion culture |
| Tangential flow | Feed strategy |
| Batch | |

For example, the production parameter can be culturing a cell under acidic, neutral or basic pH conditions. Temperatures can be selected from 10 to 42°C. For example, a temperature of about 28 to 36°C does not significantly alter galactosylation, fucosylation, high mannose production, hybrid production or sialylation of glycoproteins produced by a cell (e.g., a CHO cell, e.g., a dhfr deficient CHO cell) cultured at these temperatures. In addition, any method that slows down the growth rate of a cell may also have this effect. Thus, temperatures in this range or methods that slow down growth rate can be selected when it is desirable not to have this parameter of production altering glycosynthesis.

In other instances, carbon dioxide levels can be selected which results in a desired glycan characteristic or characteristics. CO₂ levels can be, e.g., about 5%, 6%, 7%, 8%, 9%, 10%, 11%, 13%, 15%, 17%, 20%, 23% and 25% (and ranges in between). In one instance, when decreased fucosylation is desired, the cell can be cultured at CO₂ levels of about 11 to 25%, e.g., about 15%. CO₂ levels can be adjusted manually or can be a cell byproduct.

A wide array of flasks, bottles, reactors, and controllers allow the production and scale up of cell culture systems. The system can be chosen based, at least in part, upon its correlation with a desired glycan property or properties.

Cells can be grown, for example, as batch, fed-batch, perfusion, or continuous cultures.

Production parameters that can be selected include, e.g., addition or removal of media including when (early, middle or late during culture time) and how often media is harvested; increasing or decreasing speed at which cell cultures are agitated; increasing or decreasing temperature at which cells are cultured; adding or removing media such that culture density is adjusted; selecting a time at which cell cultures are started or stopped; and selecting a time at which cell culture parameters are changed. Such parameters can be selected for any of the batch, fed-batch, perfusion and continuous culture conditions, e.g., described below.

*Batch Culture:* Batch culture is carried out by placing the cells to be cultured into a fixed volume of culture medium and allowing the cells to grow. Cell numbers increase, usually exponentially, until a maximum is reached, after which growth becomes arrested and the cells die. This may be due either to exhaustion of a nutrient or accumulation of an inhibitor of growth. To recover product, cells are removed from the medium either when the cells have died or at an earlier, predetermined point. Batch culture is characterized in that it proceeds in a fixed volume (since nothing is added after placing the cells in the medium), for a fixed duration (dependent on the length of time the cells survive) with a single harvest and with the cells dying or being discarded at the end of the process.

*Fed-Batch Culture:* This is a variation on batch culture and involves the addition of a feed to the batch. Cells are cultured in a medium in a fixed volume. Before the maximum cell concentration is reached, specific supplementary nutrients are added to the culture. The volume of the feed is minimal compared to the volume of the culture. A fed-batch culture involves a batch cell culture to which substrate, in either solid or concentrated liquid form, is added either periodically or continuously during the period of growth. Fed batch culture is also characterized in that it usually proceeds in a substantially fixed volume, for a fixed duration, and with a single harvest either when the cells have died or at an earlier, predetermined point. Fed-batch cultures are described, e.g., in U.S. Pat. Nos. 5,672,502.

*Perfusion Culture:* In a perfusion culture, medium is perfused through the reactor at a high rate while cells are retained or recycled back into the reactor by sedimentation, centrifugation or filtration. Up to ten reactor volumes of medium is perfused through the bioreactor in a day. The major function of perfusing such a large volume of medium is primarily to remove the metabolites, mainly lactate, from the culture fluid. Perfusion cultures are described, e.g., in U.S. Pat. No. 6,544,788.

*Continuous Culture:* In continuous culture, the cells are initially grown in a fixed volume of medium. To avoid the onset of the decline phase, a pumped feed of fresh medium is initiated before maximum cell concentration is reached. Culture, containing a proportion of the cells, is continuously removed from the vessel to maintain a constant volume. The process removes a product, which can be continuously harvested, and provides a continuous supply of nutrients, which allows the cells to be maintained in an exponentially growing state. Continuous culture is characterized by a continuous increase in culture volume, of product and maintenance of exponentially growing culture. There is little or no death or decline phase. In a continuous culture, cells are continuously fed fresh nutrient medium, while spent medium, cells, and excreted cell product are continuously drawn off. Continuous cultures and bioreactors are described, e.g., in U.S. Pat. Nos. 4,764,471; 5,135,853; 6,156,570.

### Bioreactors

A bioreactor is a device or system that supports a biologically-active environment, e.g., a device or system meant to grow cells or tissues in the context of cell culture (e.g., mammalian, plant, yeast, bacterial cells). This process can either be aerobic or anaerobic. Bioreactors are commonly cylindrical, ranging in size from some liter to cube meters, and are often made of stainless steel. On the basis of mode of operation, a bioreactor may be classified as batch, fed batch or continuous (e.g. continuous stirred-tank reactor model).

A bioreactor can be used for large culture volumes (in the range 100-10,000 liters). Suspension cell lines can be kept in suspension, e.g., by a propeller in the base of the chamber vessel (e.g., stir tank or stir flask bioreactors) or by air bubbling through the culture vessel. Both of these methods of agitation can give rise to mechanical stresses. Membranes, porous matrices (e.g., ceramic matrices), and polysaccharide gels can be used to protect cells from shear and/or to obtain high cell densities in bioreactors that are productive for periods of weeks or months.

Rotary bioreactors use rolling action to keep cells well perfused, akin to roller bottles. In order to create a high-density environment, the culture chamber can be separated from the feeder chamber by a semipermeable membrane. This allows media to be changed without disturbing the cells. Using this principle, the rotating action in Synthecon's Rotary Cell Culture System (RCCS) creates a microgravity environment, virtually eliminating shear forces. This allows the cell to shift resources from damage control to establishing relationships with other cells, mimicking the complex three-dimensional (3-D) matrices found in vivo. Reactor vessels come in sizes ranging from 10 ml to 500 ml.

Non-limiting examples of bioreactors are as follows.

The Heraeus miniPERM bioreactor combines an autoclavable outer nutrient container and a disposable inner bioreactor chamber. The appropriate molecular weight cut-off membrane for a desired product (e.g.,. a product described herein) can be selected. Its small size allows it to fit inside standard incubators. Densities greater than 10⁷ cells per ml and product yields of 160 mg in four weeks are possible.

New Brunswick Scientific's CELLIGEN PLUS® is a highly flexible system for culture of virtually all eukaryotic cell lines. Features include a double screen impeller for increased O₂ saturation, interactive four-gas control, internal ring sparger, five programmable pumps, computer interface for system control and data logging, and four-channel recorder output. The unit may be used either as a stir tank or fibrous-bed system.

The Wave Bioreactor™ (from Wave Biotech, LLC) employs an adjustable-speed rocking platform and electric air pump to gently aerate the culture while keeping shear forces low. Smaller cultures and rocking platforms will fit in a standard incubator. Culture medium and cells only contact a presterile, disposable chamber called a cellbag that is placed on a special rocking platform. The rocking motion of this platform induces waves in the culture fluid. These waves provide mixing and oxygen transfer, resulting in a perfect environment for cell growth that can easily support over 20 x 10⁶ cells/ml. The bioreactor requires no cleaning or sterilization, providing the ultimate ease in operation and protection against cross-contamination.

Quark Enterprises provides a full range of bioreactors including its Spingro® flasks for high-density culture. These borosilicate stir flasks range from 100 ml to 36 1 and feature Teflon® spin paddles, side vents for probes and easy sampling, and jacketed models for use with a recirculating water bath. All models are completely autoclavable.

The ProCulture DynaLift system (Corning) facilitates perfusion and reduces shear effects by using an extended paddle, side baffles, and bottom contours. It is available in a range of sizes, from 125 ml to 36 1.

Another example is Braun Biotech's Biostat® Bioreactor.

The largest cultures of cells have often been achieved in fermenter-type systems. Suspension cells are most direct to scale up in this system. Cell growth and harvesting is often straightforward once the parameters for achieving maximum product have been delineated. In-line monitors for pH, gas saturation, and metabolites are available from most suppliers. Adherent cells pose more of a challenge. Some can be "suspension-adapted." Microcarrier beads as a support can be employed to improve culturing (see below).

### Stir Tank Bioreactors:

Stir tanks (and flasks) can provide cell cultures with increased density. Examples include the following.

A disposable Stirred Tank Bioreactor (Xcellerex): a scaleable, disposable stir tank bioreactor (XDR™) that can operate as a stand-alone skid mounted system or is integrated into a FlexFactory™. The XDR incorporates process sensors that monitor and control the culture conditions up to 1,000Lor 2,000L working volume scale. FlexFactory™ is a complete, turnkey, modular production train for biotherapeutics and vaccines. The single-use, disposable components that are central to the FlexFactory™, provide it with great flexibility to accommodate new process changes, including production of multiple products at a single site, and to establish manufacturing capacity rapidly, at dramatically lower costs than traditional fixed-tank, hard-piped facilities.

Applikon offers a full line of stir tanks, from 2.3 1 bench systems to 10,000 1 production units. Pumps, probes, controllers, and software are also available for all units. Borosilicate glass vessels are available up to 20 L and can be fitted with lip-sealed or magnetically coupled stirrers. Stainless steel BioClave™ vessels are designed for moderate to large-scale production and feature a flush-mounted longitudinal sight glass as well as a choice of lip-seal or magnetic stirrers.

### Airlift Bioreactors:

An alternative to the stirred tank is the airlift bioreactor. The reactor has no moving blades to create shear forces, which some mammalian and hybridoma cells are particularly sensitive to. Media perfuse from the top while oxygen enters through the bottom, creating a near-ideal mixing environment.

Kimble-Kontes manufactures the CYTOLIFT® glass airlft bioreactor with an effective volume of 580 ml. It is easily cleaned and fully autoclavable for consistent performance and long life. A glass jacket is standard on all models. Other features include a check valve to prevent backflow in case of pressure drop, vent, infusion and effluent ports, plus three ports for pH, foam level, and dO2 probes. CYTOSTIR® (also from Kimble-Kontes) is a line of double-sidearm bioreactors in nine sizes, from 100 ml to 36 l. The large, height-adjustable stirring blades are constructed of TEFLON® to minimize cell adhesion and facilitate cleaning. Components are steam-autoclavable.

### Batch Bioreactors:

In batch bioreactors, the medium and inoculum are loaded in the beginning and the cells are allowed to grow. There is no addition/replacement of medium, and the entire cell mass is harvested at the end of incubation period. The characteristic features of such bioreactor systems are as follows: (i) continuous depletion of medium, (ii) accumulation of cellular wastes, (iii) alterations in growth rate and (iv) continuous change in the composition of cells.

A spin filter bioreactor can be used as a batch bioreactor by closing the inlet for medium and the outlets for medium/medium plus cells.

Batch bioreactors are available, e.g., from Rockland Immunochemicals, Inc.

### Fed-Batch Bioreactors:

In fed-batch (semi-batch) reactors, feed is added, but effluent (and cells) are not removed. Thus fed-batch reactors can be used to maintain cells under low substrate or nutrient conditions without washout occurring. Because cells are not removed during the culturing, fed-batch biorecators are well suited for the production of compounds produced during very slow or zero growth. Unlike a continuous bioreactor, the feed does not need to contain all the nutrients needed to sustain growth. The feed may contain only a nitrogen source or a metabolic precursor.

### Continuous Bioreactors:

In continuous bioreactors, there is continuous inflow of fresh medium and outflow of used medium (with or without cells) during the entire incubation period. The cells thus continuously propagate on the fresh medium entering the reactor and at same time, products, metabolic waste products and cells are removed in the effluent. A spin filter bioreactor is an example of continuous flow bioreactor. It can have the following features: (1) The central shaft of bioreactor houses a spinning, filter, which enables the removal of used medium, free of cells, through the shaft; (2) A stirrer plate magnetically coupled to the central shaft provides continuous stirring; the spinning filter also stirs the culture; (3) The culture is aerated by a sparger, which allows a wide range of aeration rates; (4) A port is provided for addition of fresh medium, while (5) another port enables removal of the culture (used medium + cells) as per need.

This bioreactor provides a highly versatile system for control on medium change rate and on cell density; this becomes possible due to the two routes for medium removal, while only one of them allows the removal of cells.

A continuous flow bioreactor can be used to grow cells at a specified cell density in an active growth phase; such cultures may either provide inocula for further culture or may serve as a continuous source of biomass yields.

### Immobilized Cell Bioreactors:

These bioreactors are based on cells entrapped either in gels, such as, agarose, agar, chitosan, gelatine, gellan, polyacrylamide and calcium alginate, to produce beads, or in a membrane or metal (stainless steel) screen compartment or cylinder.

As an example of the operation of such a bioreactor: the membrane screen cylinder containing cells is kept in a chamber through which the medium is circulated from a recycle chamber. The medium flows parallel to the screen cylinder and diffuses across the screen into the cell mass.

Similarly, products from cells diffuse into the medium and out of the screen cylinder. The membrane/screen compartment housing the cells may be cylindrical or flat, and medium movement may be so adjusted as to flow across the screen compartment rather than parallel to it. Fresh medium is regularly added to and equivalent volume of used medium is withdrawn from the recycling chamber to maintain its nutrient status.

Cell immobilization changes the physiology of cells as compared to that of cells in suspension. This technique is useful where the biochemical of interest is excreted by the cells into the medium.

Product excretion may also be brought about by immobilization itself, or by certain treatments like altered pH, use of DMSO (dimethyl sulfoxide) as a permeabilizing agent, changed ionic strength of medium, an elicitor, etc.

Immobilized cell reactors can have the following advantages: (i) no risk of cell wash out, (ii) low contamination risk, (iii) protection of cells from liquid shear, (iv) better control on cell aggregate size, (v) separation of growth phase (in a batch/continuous bioreactor) from production stage (in an immobilized cell bioreactor), (vi) cellular wastes regularly removed from the system, and (vii) cultures at high cell densities.

### Multistage Bioreactors:

Such culture systems use two or more bioreactors in a specified sequence, each of which carries out a specific step of the total production process. The simplest situation would involve two bioreactors. For example, for the production of a biochemical, both the bioreactors can be of batch type: the first bioreactor provides conditions for rapid cell proliferation and favors biomass production, while the second bioreactor has conditions conducive for biochemical biosynthesis and accumulation. The cell biomass is collected from the first stage bioreactor and is used as inoculum for the second stage reactor. As another example, the first reactor may be in continuous mode, while the second may be of batch type.

The cell mass from this bioreactor serves as a continuous source of inoculum for the second stage batch type bioreactor, which has conditions necessary for embryo development and maturation (but not for cell proliferation). The use of continuous first stage bioreactor can offer one or more advantages, e.g.: (i) avoids the time, labor and cost needed for cleaning, etc. of a batch reactor between two runs, (ii) eliminates the lag phase of batch cultures, and (iii) provides a more homogeneous and actively growing cell population.

### Perfusion Bioreactors:

### Bioreactors are available for perfusion cultures. Examples are as follows.

The CellCube® System from Corning Life Sciences provides a fast, simple, and compact method for the mass culture of attachment dependent cells in a continuously perfused bioreactor. The system is an easily expandable system for growing adherent cells in all levels of biomass, viral, and soluble biomolecule production. The basic system uses disposable CellCube®Modules with from 8,500cm² to 85,000cm² cell growth surface using the same control package. CellCube®Modules have polystyrene growth surfaces that are available with either the stand tissue culture surface or the advanced Corning CellBIND® Surface for improved cell attachment. These disposable polystyrene modules hold 3.5 l of media and contain 25 parallel plates for a total growing area of 21,000 cm2 per cube, expandable up to 340,000 cm2 (the 4/100 stack). The interlinkable cubes stand on one corner with media entering the bottom and exiting the top. The CellCube® System is comprised of four pieces of capital equipment - the system controller, oxygenator, circulation and media pumps. The digital controller features in-line monitoring of perfusion, pH, dO₂, and temperature.

### Centrifugal Bioreactors

Another type of bioreactor is a centrifugal bioreactor, e.g., Kinetic Biosystems' CBR 2000 centrifugal bioreactor. Designed for industrial production, it can achieve densities up to 10,000 times greater than stirred tank bioreactors. Media are fed in through the axle, then forced to the outside by the rotating action where they enter the reaction chamber. Cells are held in suspension by opposing centrifugal force with perfusion. Waste products are removed through the axle and sampled 10 times per hour. Real-time analysis of growth and production parameters means that any perturbation can be adjusted quickly. The end result can be increased product yield and quality. Each chamber is capable of producing 1x10¹⁶ cells with each rotor holding three chambers.

### Microcarrier

For attached cell lines (e.g., for bioreactor cell culturing), the cell densities obtained can be increased by the addition of micro-carrier beads. These small beads are 30-1005µm in diameter and can be made, e.g., of dextran, cellulose, gelatin, glass or silica, and can increase the surface area available for cell attachment. The range of micro-carriers available means that it is possible to grow most cell types in this system.

Particles come in two forms: solid and porous. Solid beads are the most manageable for biomass harvest, while porous beads are better suited for secreted or lysate products. Other matrices hold beads stationary, creating a solid bed through which media are perfused.

Microcarrier cultures using suspended macroporous beads are readily scaled up. These systems are distinct from conventional surface microcarrier culture in that the cells are immobilized at high densities inside the matrix pores and are protected from the fluidshear. Another advantage of macroporous beads is that they can be inoculated directly from the bulk medium in the same fashion as conventional microcarriers. Suspended bead immobilization systems can be used in a number of different reactor configurations including suspended beds or stirred tank bioreactors. These systems can be scaled up by increasing the volume of the bioreactor and the number of beads. Suspended macroporous bead technologies are also available. In an attempt to mimic the cell culture environment in mammals, these macroporous beads can be collagen-based (e.g., collagen, gelatin, or collagen-glycosaminoglycan).

For example, Porous ImmobaSil microbeads produced by Ashby Scientific are available in different shapes and sizes for easy adaptation to your particular culture vessel. They are gas permeable, allowing culture densities to reach 3x10⁶/ml for maximum product yield.

Amersham Pharmacia (AP) Biotech offers microcarriers and fluid-bed reactors. Cytopore I beads are optimized for CHO-type cells, while Cytopore II is for adherent cells requiring higher surface-charge density. Cytoline I beads are suited for resilient cells requiring high circulation rates. The low-density Cytoline II carrier is optimized for shear-sensitive cells such as hybridomas needing slower circulation. AP Biotech has designed the Cytopilot fluid-bed system perfusion reactor for use with its Cytoline beads.

Glass-surface microcarrier for growth of cell cultures are described in U.S. Pat. No. 4,448,884.

Further, the CYTOSTIR® line (from Kontes) of double sidearm stirred bioreactors for microcarrier cell culture has been completely redesigned to improve performance and enhance interchangeability. CYTOSTIR® bioreactors are available in nine sizes ranging from 100 mL to 36 liters. The borosilicate glass flasks have two large sidearms with screw cap closures that allow easy sampling. The dome in the center of the flask base prevents microcarriers from accumulating directly under the stirring blades. The large, height adjustable TEFLON® stirring blades are designed to provide maximum stirring efficiency to keep microcarriers in suspension at the slow stirring speeds required for tissue culture. During stirring, cultures contact only borosilicate glass and TEFLON®. All one liter and larger size flasks have anti-drip pour lips and polypropylene caps with sealing rings. All CYTOSTIR® bioreactors and components are completely steam autoclavable.

### Spinner Culture

This is a common culture method for suspension lines including hybridomas and attached lines that have been adapted to growth in suspension. Spinner flasks are either plastic or glass bottles with a central magnetic stirrer shaft and side arms for the addition and removal of cells and medium, and gassing with CO2-enriched air. Inoculated spinner flasks are placed on a stirrer and incubated under the culture conditions appropriate for the cell line. Cultures can be stirred, e.g., at 100-250 revolutions per minute. Spinner flask systems designed to handle culture volumes of 1-12 liters are available from Techne, Sigma, and Bellco, e.g. (Prod. Nos. Z380482-3L capacity and Z380474-1L capacity). Another example of spinner culture systems is the MantaRay single-use spinner flask.

Wheaton Science Products offers scale-up systems for all levels of production. Its Magna-Flex® Spinner Flasks have bulb-shaped, flex-type glass impellers for use with microbeads. A removable stainless steel pin immobilizes the impeller to prevent cell damage during handling. Available in a range of sizes from 125 ml to 8 1, they are fully autoclavable. Also available are the Cell Optimizer™ System for determination of optimum culture conditions prior to scale-up, and the OVERDRIVE™ for economical industry-level production up to 45 l.

The SuperSpinner from B. Braun Biotech is an entry-level stir flask that accommodates 500 and 1000 ml cultures and features a bubble-free aeration/agitation system. The Biostat®series of stir vessels handles culture sizes from 50 ml to 10 l and include complete ready-to-use systems and systems that integrate preexisting components.

Techne UK offers a complete line of stir flasks in volumes up to 5 l. Designed with a stirring rod rather than paddles, they simplify cleaning and autoclaving by eliminating rotating bearings. The unique stirring action creates vertical and horizontal flow in a gentle spiral throughout the culture. Its line of programmable stirring platforms features the SOFTSTART™ acceleration/deceleration control to reduce cell damage from excessive turbulence.

Wheaton Science Products offers scale-up systems for all levels of production. Its Magna-Flex® Spinner Flasks have bulb-shaped, flex-type glass impellers for use with microbeads. A removable stainless steel pin immobilizes the impeller to prevent cell damage during handling. Available in a range of sizes from 125 ml to 8 l, they are fully autoclavable. Also available are the Cell Optimizer™ for determination of optimum culture conditions prior to scale-up, and the OVERDRIVE™ for economical industry-level production up to 45 l.

### T Flask Culture

Adherent or suspension cultures can be grown in T flasks, e.g., T-25, T-76, T-225 flasks. The caps can be plug sealed or vented. The flasks can be plastic or glass. The surface of the flasks can be coated, e.g., with hydrophilic moieties that contain a variety of negatively charged functional groups and/or nitrogen-containing functional groups that support cell attachment, spreading, and differentiation. T flasks are available, e.g., from Nunc, Nalgene, Corning, Greiner, Schott, Pyrex, or Costar.

### Cell Culture Dishes

Cells can be grown in culture dishes. The surface of the dishes can be coated, e.g., with hydrophilic moieties that contain a variety of negatively charged functional groups and/or nitrogen-containing functional groups that support cell attachment, spreading, and differentiation. Dishes are available, e.g., from BD Biosciences, Corning, Greiner, Nunc, Nunclon, Pyrex.

### Suspension Cell Culture

Suspended cells can be grown, e.g., in bioreactors, dishes, flasks, or roller bottles, e.g., described herein.

### Stationary Suspension Culture Systems

An example of a stationary suspension system is CELLine™ 1000. The CELLine™ 1000 (Integra Bioscience, Chur, Switzerland) device is a membrane-based disposable cell culture system. It is composed of two compartments, a cultivation chamber (20 mL) and a nutrient supply compartment (1000 mL) separated by a semipermeable dialysis membrane (10 kD molecular weight cut-off), which allows small nutrients and growth factors to diffuse to the production chamber. Oxygen supply of the cells and CO₂ diffusion occur through a gas-permeable silicone membrane. Antibodies concentrate in the production medium. This culture system requires a CO₂ incubator. For example, for optimal production levels, the device can be inoculated with 50x10⁶ cells, and 80% of the production medium and the entire nutrition medium changed twice a week.

### Rotation Suspension Culture Systems

Such systems include roller bottles (discussed herein). An example of a rotation suspension system is the miniPERM (Vivascience, Hannover, Germany) which is a modified roller bottle two-compartment bioreactor in which the production module (35 mL) is separated from the nutrient module (450 mL) by a semipermeable dialysis membrane. Nutrients and metabolites diffuse through the membrane, and secreted antibodies concentrate in the production module. Oxygenation and CO₂ supply occur through a gas-permeable silicone membrane at the outer side of the production module and through a second silicone membrane extended into the nutrition module. The miniPERM must be placed on a roller base inside a CO₂ incubator. It is possible to place two roller bases together in a 180-L CO2 incubator, each holding a maximum of four bioreactors (i.e., the same amount of space is occupied for 1-4 incubations).

### Roller Bottle

This is the method most commonly used for initial scale-up of attached cells also known as anchorage dependent cell lines. Roller bottles are cylindrical vessels that revolve slowly (between 5 and 60 revolutions per hour) which bathes the cells that are attached to the inner surface with medium. Roller bottles are available typically with surface areas of 1050cm2 (Prod. No. Z352969). The size of some of the roller bottles presents problems since they are difficult to handle in the confined space of a microbiological safety cabinet. Recently roller bottles with expanded inner surfaces have become available which has made handling large surface area bottles more manageable, but repeated manipulations and subculture with roller bottles should be avoided if possible. A further problem with roller bottles is with the attachment of cells since as some cells lines do not attach evenly. This is a particular problem with epithelial cells. This may be partially overcome a little by optimizing the speed of rotation, generally by decreasing the speed, during the period of attachment for cells with low attachment efficiency.

Roller bottles are used in every conceivable application. A good starting point for small labs with periodic scale-up needs, they are also being used for large-scale industrial production. Because the cultures are seeded and maintained in a manner similar to flasks, typically no additional training is necessary. Small racks fit inside standard incubators, eliminating the need for additional capital expenditures.

Roller bottles come in a number of configurations: plastic, glass, pleated, flat, vented, or solid. Glass can be sterilized and reused, whereas different plastics and coatings optimize growth for an assortment of cell types. Pleats increase the effective growth surface, thereby increasing product yield without additional space requirements. Vented caps are used for culture in a CO₂ environment, while solid caps are best for culturing in a warm room or unregulated incubator. Roller bottles are available, e.g., from Corning.

### Adherent Cell Culture

Adherent cells can be grown, e.g., in bioreactors, dishes, flasks, or roller bottles, e.g., described herein. The surfaces to which the cells adhere can be treated or coated to promote or support cell attachment, spreading, and/or differentiation. Coatings include lysine (e.g., poly-D-lysine), polyethyleneimine, collagen, glycoprotein (e.g., fibronectin), gelatin, and so forth.

### Shaker Flask

Shaker flasks can be used to provide greater agitation of cell cultures to improve oxygen or gas transfer, e.g., as compared to stationary cultures. Shaker flasks are available, e.g., from Pyrex and Nalgene.

### Perfusion

Perfusion systems allow for continuous feeding of the cell chamber from external media bottle, as described herein. Cells are retained in the cell chamber (e.g., bioreactor, bed perfusion bioreactor, packed bed perfusion bioreactor). Suppliers of perfusion systems include DayMoon Industries, Inc., and New Brunswick Scientific.

### Hollow Fiber Cell Culture

Hollow fibers are small tube-like filters with a predefined molecular weight cutoff. Large bundles of these fibers can be packed into cylindrical modules, which provide an absolute barrier to cells and antibodies while ensuring perfusion of the liquid. Hollow fiber modules can provide a large surface area in a small volume. The walls of the hollow fibers serve as semipermeable ultrafiltration membranes. Cells are grown in the extracapillary space that surrounds the fibers, and medium is perfused continuously inside the fibers. Metabolites and small nutrients freely perfuse between extra- and intracapillary space according to concentration gradients. Culture monitoring can be performed by lactate measurement.

Example of such systems include: Cellex Biosciences' AcuSyst hollow fiber reactor.

Another example is the Cell-Pharm® system 100 (CP100, BioVest, Minneapolis, MN) which is a fully integrated hollow fiber cell culture system. The cell culture unit consists of two cartridges: one that serves as a cell compartment and the other, as an oxygenation unit. The system is a freestanding benchtop system with a disposable flowpath with yields of up to 400 mg/month.

The Cell-Pharm® system 2500 (CP2500, BioVest) is a hollow fiber cell culture production system that can produce high-scale quantities of a cell-produced product, e.g., of monoclonal antibodies. Unlike CP100, it consists of two fiber cartridges for the cells and hence offers a large cell growth surface (3.25 m2). A third cartridge serves for oxygenation of the medium.

The FiberCell™ (Fibercell Systems Inc., Frederick, MD) hollow-fiber cell culture system is composed of a culture medium reservoir (250 mL) and a 60-mL fiber cartridge (1.2 m2), both connected to a single microprocessor-controlled pump. It is possible to prolong the media supply cycles by replacing the original medium reservoir with a 5-L flask. In contrast to the Cell-Pharm® systems, the FiberCell™ bioreactor is used inside a CO₂ incubator. Oxygenation occurs by a gas-permeable tubing.

Cellex Biosciences makes hollow fiber reactors for all levels of production. The AcuSyst-XCELL® is designed for large-scale production of secreted proteins, producing 60 to 200 grams of protein per month. Its AcuSyst miniMax™ is a flexible research scale benchtop bioreactor capable of producing up to 10 g of protein per month. For single-use or pilot studies of a few weeks' duration, the economical RESCU-PRIMER™ produces up to 200 mg per month with a choice of hollow fiber and ceramic matrices.

The Unisyn Cell-Pharm® MicroMouse™ is a disposable system with a footprint of 1.5 ft2, fitting inside a standard lab incubator. It is capable of producing up to 250 mg of monoclonal antibodies per month for three months.

The TECNOMOUSE® by Integra Biosciences is a modular rack system with five separate cassette chambers. Up to five different cell lines can be cultivated for up to 30 weeks, each producing 200 mg of antibodies per month. The integrated gas supply and online monitoring capabilities help to control culture conditions.

### Cell Factories

Cell factories are used for large scale (e.g., industrial scale) cell culture and products of biomaterials such as vaccines, monoclonal antibodies, or pharmaceuticals. The factories can be used for adherent cells or suspension culture. The growth kinetics are similar to laboratory scale culture. Cell factories provide a large amount of growth surface in a small area with easy handling and low risk of contamination. A cell factory is a sealed stack of chambers with common vent and fill ports. A 40-chamber factory can be used in place of 30 roller bottles. Openings connecting the chambers cause media to fill evenly for consistent growth conditions. Vents can be capped or fitted with bacterial air vents. Cell factories can be molded from e.g., polystyrene. Suppliers of cell factories include Nunc. The surface of the factories can be treated to improve growth or cell attachment conditions, e.g., treated with Nunclon®Δ.

Nunclon® Cell Factories™ are low-profile, disposable, polystyrene, ventable chambers that come in stacks of one, two, 10, and 40. Inoculation, feeding, and harvest are straightforward due to the innovative design of the connected plates.

### Cell Culture Bags

Cell culture bags, e.g., single use cell culture bags, can be used for growing mammalian, insect, and plant cells. The bags convenience and flexibility for suspension, perfusion, and microcarrier culture. Suppliers of cell culture bags include DayMoon Industries, Inc. and Dunn Labortechnik GmbH.

Gentle wave motion induced by agitation of the bags creates an excellent mixing and oxygenation environment for cell growth. Equipped with internal dip-tube and mesh filter, media exchange and perfusion culture with microcarriers is simplifies. A built-in screw-cap port can provide convenience for unrestricted access of microcarrier beads, cell attachment matrix and tissue cultures.

The bag system also offers a greater flexibility in gas transfer between the bag headspace and the environment, and it is capable of both gas diffusion and continuous gas flush. Gas diffusion through the built-in microporous membrane on the screw-cap provides sufficient gas exchange for most cell culture need. If required, pressurized air or gas under 1.5 psi can be added through one of the luer ports and vented out through the membrane cap.

As an example, Optima™ is a single-use cell culture bag that offers convenience, capacity and flexibility for growing insect, plant and mammalian cells. Optima™ is designed for use on conventional laboratory shakers or rocking platforms. Available in two standard bags with working capacities up to 4 1, the Optima™ is useful for high volume suspension culture, providing a cost-effective alternative to stirred bioreactors. Optimamini™ bags are designed to fit most laboratory shakers and rocking platforms, requiring no specialized equipment.

### Purification of Glycans and Glycoproteins

Production parameters including purification and formulation can be used to produce a glycoprotein preparation with a desired glycan property or properties. Various purification processes can be used to prejudice the glycan characteristics of the purified glycoprotein preparation. For example, affinity based methods, charged based methods, polarity based methods and methods that distinguish based upon size and/or aggregation can be selected to provide a glycoprotein preparation with a desired glycan property or properties. For example, normal phase liquid chromatography can be used to separate glycans and/or glycoproteins based on polarity. Reverse-phase chromatography can be used, e.g., with derivatized sugars. Anion-exchange columns can be used to purify sialylated, phosphorylated, and sulfated sugars. Other methods include high pH anion exchange chromatography and size exclusion chromatography can be used and is based on size separation.

Affinity based methods can be selected that preferentially bind certain chemical units and glycan structures. Matrices such as m-aminophenylboronic acid, immobilized lectins and antibodies can bind particular glycan structures. M-aminophenylboronic acid matrices can form a temporary covalent bond with any molecule (such as a carbohydrate) that contains a 1,2-cis-diol group. The covalent bond can be subsequently disrupted to elute the protein of interest. Lectins are a family of carbohydrate-recognizing proteins that exhibit affinities for various monosaccharides. Lectins bind carbohydrates specifically and reversibly. Primary monosaccharides recognized by lectins include mannose/glucose, galactose/N-acetylgalactosamine, N-acetylglucosamine, fucose, and sialic acid (QProteome Glycoarray Handbook, Qiagen, September 2005, available at:
http://wolfson.huji.ac.il/purification/PDF/Lectins/QIAGEN_GlycoArraHandbook.pdf) or similar references. Lectin matrices (e.g., columns or arrays) can consist of a number of lectins with varying and/or overlapping specificities to bind glycoproteins with specific glycan compositions. Some lectins commonly used to purify glycoproteins include concavalin A (often coupled to Sepharose or agarose) and Wheat Germ. Anti-glycan antibodies can also be generated by methods known in the art and used in affinity columns to bind and purify glycoproteins.

The interaction of a lectin or antibody with a ligand, such as a glycoprotein, allows for the formation of cross-linked complexes, which are often insoluble and can be identified as precipitates (Varki et al., ed., "Protein-Glycan Interactions" in Essentials of Glycobiology available at world wide web at http://www.ncbi.nlm.nih.gov/books/bv.fcgi?rid=glyco.section.269) or similar references. In this technique, a fixed amount of lectin or antibody (receptor) is titrated with a glycoprotein or a glycan, and at a precise ratio of ligand to receptor, a precipitate is formed (Varki et al.). Such precipitation is highly specific to the affinity constant of the ligand to the receptor (Varki et al.). Another precipitation approach takes advantage of the fact that a complex between a lectin and a glycan can be "salted" out or precipitated by ammonium sulfate (Varki et al.).

### Target Glycoprotein Product

Methods described herein can be used to provide a target glycoprotein product having a desired glycan property or properties. As described herein, the glycan property or properties of the target glycoprotein product can be the same or substantially similar to a primary glycoprotein product or the glycan property or properties of the target glycoprotein product can be different than those of the primary glycoprotein product. For example, the glycan property of the target glycoprotein product can be a glycan characteristic that differs from the glycan characteristic of a primary glycoprotein product such as the degree of heterogeneity of glycan structures attached to a preselected site. In some instances , the glycan property can be, e.g., a functional property that differs from the primary target glycoprotein product. Functional properties include, but are not limited to, serum half life, clearance, stability *in vitro* (shelf life) or *in vivo,* binding affinity, tissue distribution and targeting, toxicity, immunogenecity, absorption rate, elimination rate, and bioavailability.

A production parameter or parameters can be determined and/or selected to produce a glycoprotein product that has a different glycan characteristic or characteristics that, e.g., have been correlated with a different functional property than the primary glycoprotein product. Correlations between various glycan characteristics and functional properties which that characteristic can affect are described herein. Table III provides examples of such correlations.

| **Table III** | | |
|---|---|---|
| **Glycan** | **Functional Characterization** | **Rationale** |
| Sialic acid terminal | Bioavailability | In some instances an increase in sialylation leads to a corresponding decrease in exposed terminal galactose and subsequent increase in bioavailability |
| | Targeting | In some instances an increase in sialylation has the potential for targeting to any class of sialic acid binding lectins which may include but are not limited to the selectins (E,P, and L) and the siglecs (1-11). In some instances this may increase delivery across the blood brain barrier. |
| | Receptor affinity | In some instances a decrease in sialylation can lead to an increase in receptor affinity (e.g. decrease in charge repulsion) |
| Galactose terminal | Bioavailability | In some instances an increase in terminal galactose residues leads through decreased bioavailability (e.g.increased binding to the asiologlycoprotein receptor (or hepatic lectin) and endocytosis) |
| | Targeting | In some instances in increase in galactosylation lead to increased targeting to or complexing with galactose binding proteins which may include but are not limited to the galectins |
| | C1q | In some instances an increase in galactosylation leads to increased C1q and complement cytotoxicity |
| Alpha linked Galactose terminal | Immunogenecity | In some instances the presence of alpha linked terminal galactose leads to increased immunogenecity |
| Fucosylation | ADCC | In some instances the presence of a core fucose moiety decreases ADCC activity |
| | Targeting | In some instances the presence of a branched fucose moiety may be used to target the protein to specific lectin receptors which may include but are not limited to the selectins (E, P, and L) |
| High Mannose | Targeting | In some instances the presence of high mannose type structures (including but not limited to Man5, Man6, Man7, Man8 and Man9) can be used to target the protein to mannose specific receptors (which may include but are not limited to the macrophage mannose receptor). In some instances the presence of high mannose structures on growth factors (e.g. FGF) lead to specific distribution to kidney |
| | Receptor affinity | In some instances high-mannose structures on TSH showed the highest biopotency for signaling (e q.cAMP and IP stimulation) |
| Mannose-6-Phsophate | Targeting | In some instances the presence of mannose-6-phosphate structures can be used to target the protein to specific receptors which may include but are not limited to the mannose-6-phosphate receptor |
| | Receptor affinity | In some instances the presence of mannose-6-phosphate structures can decrease receptor affinity (e.g. through charge repulsion) |
| Sulfation | Targeting | In some instances the presence of sulfated glycans can be used to target the protein to receptors which may include but are not limited to the siglecs (1-11) and the selectins (E,P, and L) |
| | Receptor affinity | In some instances the presence of sulfated glycans can be used to regulate the affinity of the protein to its target receptor through charge based repulsion |
| N-glycolyl neuraminic acid | Immunogenecity | In some instances high levels of N-glycolyl neurmainic acid may be immunogenic |
| GlcNAc terminal | Bioavailability | In some instances increasing terminal GlcNAc residues decreases bioavailability (e.g. binding to the mannose receptor) |
| GlcNAc bisecting | Receptor affinity | In some instances increasing levels of bisecting GlcNAc increases ADCC activity |
| Site Occupancy | Receptor affinity / function | In some instances site occupancy can control receptor affinity. In some instances the degree of site occupancy can control complement mediated Ab cytotoxicity |

The amino acid sequence of the target glycoprotein product can be identical to the amino acid sequence of the primary glycoprotein product or the amino acid sequence can differ, e.g., by up to 1, 2, 3, 4, 5, 10 or 20 amino acid residues, from the amino acid sequence of the primary glycoprotein product. Proteins and fragments thereof can be glycosylated at arginine residues, referred to as N-linked glycosylation, and at serine or threonine residues, referred to as O-linked glycosylation. In some instances, the amino acid sequence of a target glycoprotein product can be modified to add a site for attaching a saccharide moiety. The amino acid sequence of the target glycoprotein product can be, e.g., modified to replace an amino acid which does not serve as a site for glycosylation with an amino acid which serves as a site for glycosylation. The amino acid sequence of the target glycoprotein product can also be modified by replacing an amino acid which serves as a site for one type of glycosylation, e.g., O-linked glycosylation, with an amino acid which serves as a site for a different type of glycosylation, e.g., an N-linked glycosylation. Further, an amino acid residue can be added to an amino acid sequence for a target glycoprotein product to provide a site for attaching a saccharide. Modification of the amino acid sequence can also be at one or more amino acid residues not associated with a potential glycosylation site. An amino acid sequence of a glycoprotein product or the nucleotide sequence encoding it, can be modified by methods known in the art.

### Exemplary Computer Implementation

The methods and articles (e.g., systems or databases) described herein need not be implemented in a computer or electronic form. A database described herein, for example, can be implemented as printed matter.

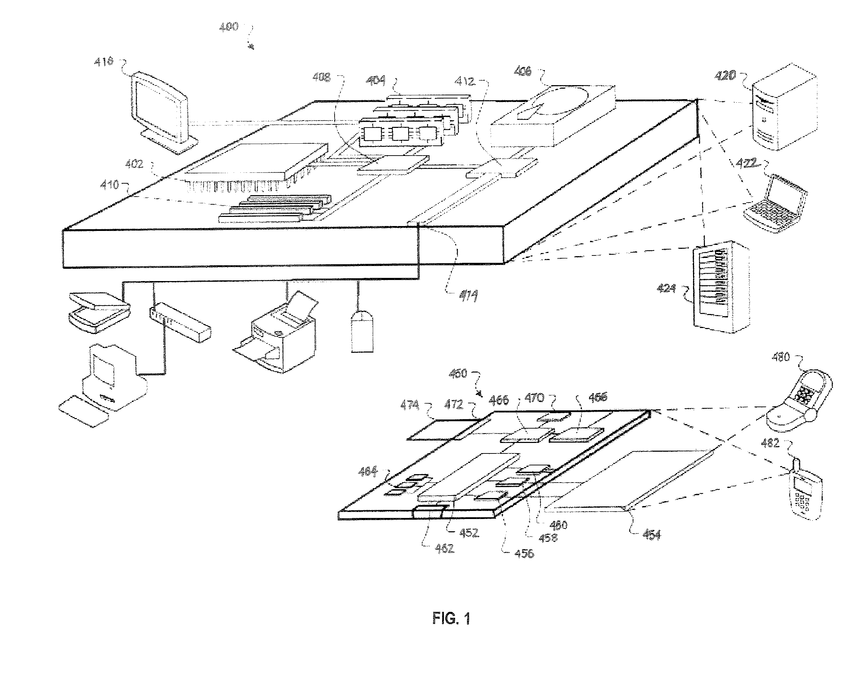

In an exemplary computer implementation, Figure 1 is a block diagram of computing devices and systems 400, 450. Computing device 400 is intended to represent various forms of digital computers, such as laptops, desktops, workstations, personal digital assistants, servers, blade servers, mainframes, and other appropriate computers. Computing device 450 is intended to represent various forms of mobile devices, such as personal digital assistants, cellular telephones, smartphones, and other similar computing devices. The components shown here, their connections and relationships, and their functions, are meant to be exemplary only, and are not meant to limit implementations described and/or claimed in this document.

Computing device 400 includes a processor 402, memory 404, a storage device 406, a high-speed interface 408 connecting to memory 404 and high-speed expansion ports 410, and a low speed interface 412 connecting to low speed bus 414 and storage device 406. Each of the components 402, 404, 406, 408, 410, and 412, are interconnected using various busses, and can be mounted on a common motherboard or in other manners as appropriate. The processor 402 can process instructions for execution within the computing device 400, including instructions stored in the memory 404 or on the storage device 406 to display graphical information for a GUI on an external input/output device, such as display 416 coupled to high speed interface 408. In other implementations, multiple processors and/or multiple buses can be used, as appropriate, along with multiple memories and types of memory. Also, multiple computing devices 400 can be connected, with each device providing portions of the necessary operations (e.g., as a server bank, a group of blade servers, or a multi-processor system).

The memory 404 stores information within the computing device 400. In one implementation, the memory 404 is a computer-readable medium. In one implementation, the memory 404 is a volatile memory unit or units. In another implementation, the memory 404 is a non-volatile memory unit or units.

The storage device 406 is capable of providing mass storage for the computing device 400. In one implementation, the storage device 406 is a computer-readable medium. In various different implementations, the storage device 406 can be a floppy disk device, a hard disk device, an optical disk device, or a tape device, a flash memory or other similar solid state memory device, or an array of devices, including devices in a storage area network or other configurations. In one implementation, a computer program product is tangibly embodied in an information carrier. The computer program product contains instructions that, when executed, perform one or more methods, such as those described above. The information carrier is a computer- or machine-readable medium, such as the memory 404, the storage device 406, memory on processor 402, or a propagated signal.

The high speed controller 408 manages bandwidth-intensive operations for the computing device 400, while the low speed controller 412 manages lower bandwidth-intensive operations. Such allocation of duties is exemplary only. In one implementation, the high-speed controller 408 is coupled to memory 404, display 416 (e.g., through a graphics processor or accelerator), and to high-speed expansion ports 410, which can accept various expansion cards (not shown). In the implementation, low-speed controller 412 is coupled to storage device 406 and low-speed expansion port 414. The low-speed expansion port, which can include various communication ports (e.g., USB, Bluetooth, Ethernet, wireless Ethernet) can be coupled to one or more input/output devices, such as a keyboard, a pointing device, a scanner, or a networking device such as a switch or router, e.g., through a network adapter.

The computing device 400 can be implemented in a number of different forms, as shown in the figure. For example, it can be implemented as a standard server 420, or multiple times in a group of such servers. It can also be implemented as part of a rack server system 424. In addition, it can be implemented in a personal computer such as a laptop computer 422. Alternatively, components from computing device 400 can be combined with other components in a mobile device (not shown), such as device 450. Each of such devices can contain one or more of computing device 400, 450, and an entire system can be made up of multiple computing devices 400, 450 communicating with each other.

Computing device 450 includes a processor 452, memory 464, an input/output device such as a display 454, a communication interface 466, and a transceiver 468, among other components. The device 450 can also be provided with a storage device, such as a microdrive or other device, to provide additional storage. Each of the components 450, 452, 464, 454, 466, and 468, are interconnected using various buses, and several of the components can be mounted on a common motherboard or in other manners as appropriate.

The processor 452 can process instructions for execution within the computing device 450, including instructions stored in the memory 464. The processor can also include separate analog and digital processors. The processor can provide, for example, for coordination of the other components of the device 450, such as control of user interfaces, applications run by device 450, and wireless communication by device 450.

Processor 452 can communicate with a user through control interface 458 and display interface 456 coupled to a display 454. The display 454 can be, for example, a TFT LCD display or an OLED display, or other appropriate display technology. The display interface 456 can comprise appropriate circuitry for driving the display 454 to present graphical and other information to a user. The control interface 458 can receive commands from a user and convert them for submission to the processor 452. In addition, an external interface 462 can be provide in communication with processor 452, so as to enable near area communication of device 450 with other devices. External interface 462 can provide, for example, for wired communication (e.g., via a docking procedure) or for wireless communication (e.g., via Bluetooth or other such technologies).

The memory 464 stores information within the computing device 450. In one implementation, the memory 464 is a computer-readable medium. In one implementation, the memory 464 is a volatile memory unit or units. In another implementation, the memory 464 is a non-volatile memory unit or units. Expansion memory 474 can also be provided and connected to device 450 through expansion interface 472, which can include, for example, a SIMM card interface. Such expansion memory 474 can provide extra storage space for device 450, or can also store applications or other information for device 450. Specifically, expansion memory 474 can include instructions to carry out or supplement the processes described above, and can include secure information also. Thus, for example, expansion memory 474 can be provided as a security module for device 450, and can be programmed with instructions that permit secure use of device 450. In addition, secure applications can be provided via the SIMM cards, along with additional information, such as placing identifying information on the SIMM card in a non-hackable manner.

The memory can include for example, flash memory and/or MRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 464, expansion memory 474, memory on processor 452, or a propagated signal.

Device 450 can communicate wirelessly through communication interface 466, which can include digital signal processing circuitry where necessary. Communication interface 466 can provide for communications under various modes or protocols, such as GSM voice calls, SMS, EMS, or MMS messaging, CDMA, TDMA, PDC, WCDMA, CDMA2000, or GPRS, among others. Such communication can occur, for example, through radio-frequency transceiver 468. In addition, short-range communication can occur, such as using a Bluetooth, WiFi, or other such transceiver (not shown). In addition, GPS receiver module 470 can provide additional wireless data to device 450, which can be used as appropriate by applications running on device 450.

Device 450 can also communication audibly using audio codec 460, which can receive spoken information from a user and convert it to usable digital information. Audio codex 460 can likewise generate audible sound for a user, such as through a speaker, e.g., in a handset of device 450. Such sound can include sound from voice telephone calls, can include recorded sound (e.g., voice messages, music files, etc.) and can also include sound generated by applications operating on device 450.

The computing device 450 can be implemented in a number of different forms, as shown in the figure. For example, it can be implemented as a cellular telephone 480. It can also be implemented as part of a smartphone 482, personal digital assistant, or other similar mobile device.

Where appropriate, the systems and the functional operations described in this specification can be implemented in digital electronic circuitry, or in computer software, firmware, or hardware, including the structural means disclosed in this specification and structural equivalents thereof, or in combinations of them. The techniques can be implemented as one or more computer program products, i.e., one or more computer programs tangibly embodied in an information carrier, e.g., in a machine readable storage device or in a propagated signal, for execution by, or to control the operation of, data processing apparatus, e.g., a programmable processor, a computer, or multiple computers. A computer program (also known as a program, software, software application, or code) can be written in any form of programming language, including compiled or interpreted languages, and it can be deployed in any form, including as a stand alone program or as a module, component, subroutine, or other unit suitable for use in a computing environment. A computer program does not necessarily correspond to a file. A program can be stored in a portion of a file that holds other programs or data, in a single file dedicated to the program in question, or in multiple coordinated files (e.g., files that store one or more modules, sub programs, or portions of code). A computer program can be deployed to be executed on one computer or on multiple computers at one site or distributed across multiple sites and interconnected by a communication network.

The processes and logic flows described in this specification can be performed by one or more programmable processors executing one or more computer programs to perform the described functions by operating on input data and generating output. The processes and logic flows can also be performed by, and apparatus can be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, the processor will receive instructions and data from a read only memory or a random access memory or both. The essential elements of a computer are a processor for executing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. Information carriers suitable for embodying computer program instructions and data include all forms of non volatile memory, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, aspects of the described techniques can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input.

The techniques can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation, or any combination of such back-end, middleware, or front-end components. The components of the system can be interconnected by any form or medium of digital data communication, e.g., a communication network. Examples of communication networks include a local area network ("LAN") and a wide area network ("WAN"), e.g., the Internet.

The computing system can include clients and servers. A client and server are generally remote from each other and typically interact through a communication network. The relationship of client and server arises by virtue of computer programs running on the respective computers and having a client-server relationship to each other.

A number of implementations have been described. Nevertheless, it will be understood that various modifications can be made without departing from the spirit and scope of the described implementations. For example, the actions recited in the claims can be performed in a different order and still achieve desirable results. Accordingly, other implementations are within the scope of the following claims.

### Other Post-Translational Modifications

Methods, databases and products are described herein primarily with reference to glycosylation but also include analogous methods in which other post-translational modifications, are addressed in the same way as glycosylation. Examples of post-translational modification that can be included are: proteolysis, racemization, N-O acyl shift, multimerization, aggregation, sugar modification, biotinylation, neddylation, acylation, formylation, myristoylation, pyroglutamate formation, methylation, glycation, carbamylation, amidation, glycosyl phosphatidylinositol addition, O-methylation, glypiation, ubiquitination, SUMOylation, methylation, acetylation, acetylation, hydroxylation, ubiquitination, SUMOylation, desmosine formation, deamination and oxidation to aldehyde, O-glycosylation, imine formation, glycation, carbamylation, disulfide bond formation, prenylation, palmitoylation, phosphorylation, dephosphorylation, glycosylation, sulfation, porphyrin ring linkage, flavin linkage, GFP prosthetic group (Thr-Tyr-Gly sequence) formation, lysine tyrosine quinone (LTQ) formation, topaquinone (TPQ) formation, succinimide formation, transglutamination, carboxylation, polyglutamylation, polyglycylation, citrullination, methylation and hydroxylation.

This invention and disclosure are further illustrated by the following examples that should not be construed as limiting.

### Examples

### Example I: Correlations Between Various Production Parameters and Glycan Properties for Production in CHO cells

Various media production parameters were studied to determine the effect, if any, adjustment of that media production parameter had on the glycan characteristics of an anti-IL-8 antibody produced in dhfr deficient CHO cells. The cells were cultured in T flasks. The results are provided below in Table VI. Table VI indicates each production parameter (Column A) and glycan characteristic (Row A) that was evaluated. The rest of the production parameters were maintained constant throughout the evaluation. Certain effects that a production parameter has on a glycan characteristic is noted.

**Table VI.**

| **A** | **Galactosylation** | **Fucosylation** | **High Mannose** | **Hybrid** | **Sialylation** |
|---|---|---|---|---|---|
| **Mannose** | | | | | |
| **Glucosamine** | Decreased | Decreased | Increased | Increased | |
| **ManNAc** | | | | | |
| **Butyrate** | Increased | | | | |
| **450 mOsm** | | Decreased | | | |
| **Ammonia** | Decreased | Decreased | Increased | | |
| **32°C** | | | | | |
| **15% CO2** | | Decreased | | | |
| **Manganese** | | Decreased | | | |
| **Glucosamine with Uridine** | | | | | |
| **Uridine** | | | Decreased | | |

Glucosamine content was evaluated at 0, 3mM, 10 mM and 20mM glucosamine content.

As the Fc portion of IgG molecules are blocked from sialylation (likely through steric hindrance from the protein backbone), little increase in sialylation was observed following supplementation with ManNAc (*), In a second example, CHO cells expressing a fusion construct CTLA4-Ig were cultured in the presence of elevated ManNAc. As this molecule is not sterically constrained, the levels of sialic acid increased significantly in the presence of elevated ManNAc.

### Example II: Correlation of non linear additive relationships between production parameters and glycan properties for production in CHO cells.

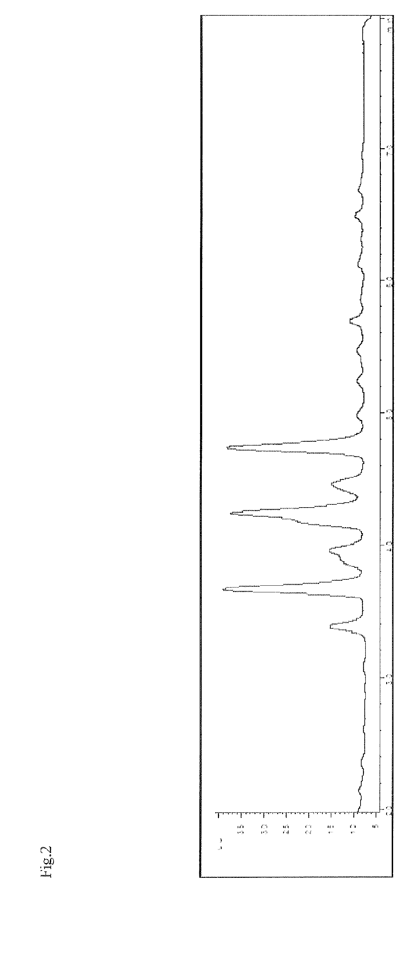

Various media production parameters were studies to determine the effect, if any, adjustment of that media production parameter had on the glycan characteristics of a human IgG antibody produced in dhfr deficient CHO cells. The cells were cultured in T flasks. Protein was then harvested, the glycans released by enzymatic digestion with Peptide:N-glycosidase F (PNGase-F) and isolated. PNGase-F is an amidase that cleaves between the innermost GlcNAc and asparagine residues of high mannose, hybrid, and complex oligosaccharides from N-linked glycoproteins (Marley et al., 1989, Anal. Biochem., 180:195). PNGase F can hydrolyze nearly all types ofN-glycan chains from glycopeptides and/or glycoproteins. The resulting glycan sample was purified using activated graphitized carbon solid phase extraction cartridges, and labeled on their reducing termini with a fluorescent tag, 2-benzamide. The labeled glycans were subsequently resolved by NP-HPLC using an amide column and their patterns determined. See Fig. 2. Glycan profiles were normalized for protein level and finally expressed as a percentage of the total glycan peak area.

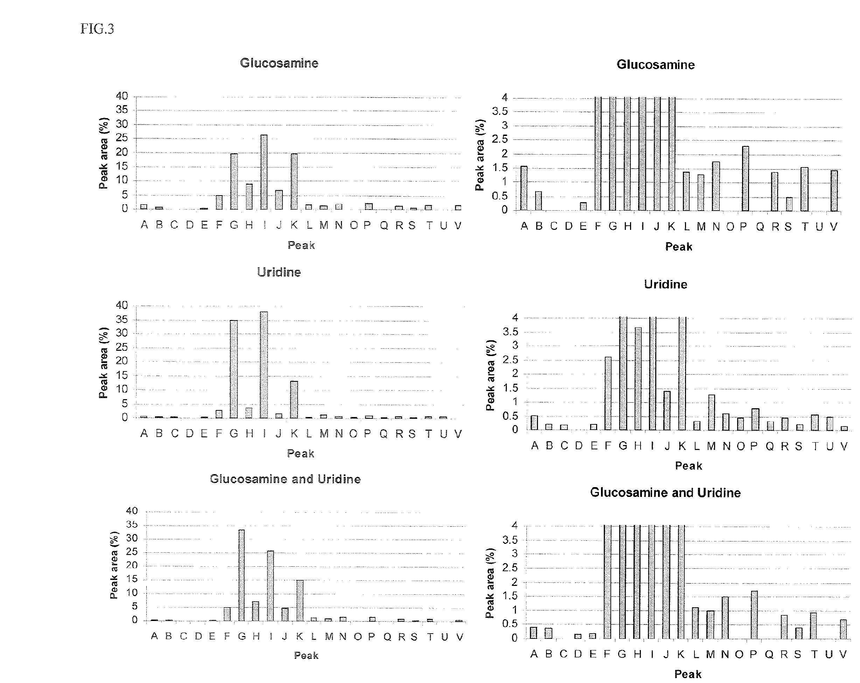

The pattern of glycans on the antibody produced in the presence of elevated glucosamine, uridine, or both uridine and glucosamine are illustrated in the Fig. 3. The glycan profile pattern observed on IgG produced in the presence of both uridine and glucosamine is not predicted from the profiles observed from an antibody produced in the presence of uridine or glucosamine individually. E.g., the relationship between the production parameters and glycan characteristics represented by peaks A, B, D, M, T and V are nonlinear.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method of producing a protein with a modulated amount of a glycan characteristic by modulating a production parameter, said method comprising:
selecting a reference level of said glycan characteristic;
selecting a value for a production parameter to provide a modulated level of said glycan characteristic; and
applying the selected value of said production parameter in a process for making a protein with a modulated amount of said glycan characteristic, wherein said process for making the protein comprises culturing a CHO cell in a cell culture medium;
wherein:
said glycan characteristic is fucosylation and said modulated amount of said glycan characteristic is a decreased level of fucosylation; and
said production parameter is the glucosamine content of the cell culture medium and said value for said production parameter is a glucosamine concentration of 0.001 to 40 mM.

2. The method of claim 1, wherein glucosamine is added to the cell culture medium.

3. The method of claim 1, wherein glucosamine is batch fed to the cell.

4. The method of claim 1, wherein an enzyme and/or substrate is added to the cell culture medium or batch fed to the cell such that glucosamine is produced.

5. The method of claim 4, wherein the substrate is selected from: N-acetylglucosamine, N-acetylglucosamine 6-phosphate, N-acetylmannosamine, and fructose.

6. The method of any one of the preceding claims, wherein the protein is an antibody.

## Patentansprüche

1. Verfahren zur Herstellung eines Proteins mit einer modulierten Menge einer Glycan-Charakteristik durch Modulieren eines Herstellungsparameters, wobei man bei dem Verfahren:
ein Referenzniveau der Glycan-Charakteristik auswählt;
einen Wert für einen Herstellungsparameter auswählt, um ein moduliertes Niveau der Glycan-Charakteristik bereitzustellen; und
den ausgewählten Wert des Herstellungsparameters bei einem Verfahren zur Erzeugung eines Proteins mit einer modulierten Menge der Glycan-Charakteristik anwendet, wobei das Verfahren zur Erzeugung des Proteins das Kultivieren einer CHO-Zelle in einem Zellkulturmedium umfasst;
wobei:
es sich bei der Glycan-Charakteristik um Fucosylierung und bei der modulierten Menge der Glycan-Charakteristik um ein vermindertes Fucosylierungsniveau handelt; und
es sich bei dem Herstellungsparameter um den Glucosamingehalt des Zellkulturmediums und bei dem Wert für den Herstellungsparameter um eine Glucosaminkonzentration von 0,001 bis 40 mM handelt.

2. Verfahren nach Anspruch 1, wobei Glucosamin zum Zellkulturmedium gegeben wird.

3. Verfahren nach Anspruch 1, wobei Glucosamin der Zelle schubweise zugeführt wird.

4. Verfahren nach Anspruch 1, wobei ein Enzym und/oder Substrat zum Zellkulturmedium gegeben oder der Zelle schubweise zugeführt wird, so dass Glucosamin produziert wird.

5. Verfahren nach Anspruch 4, wobei das Substrat aus N-Acetylglucosamin, N-Acetylglucosamin-6-phosphat, N-Acetylmannosamin und Fructose ausgewählt ist.

6. Verfahren nach einem der vorhergehenden Ansprüche, wobei es sich bei dem Protein um einen Antikörper handelt.

## Revendications

1. Procédé de production d'une protéine avec une quantité modulée d'une caractéristique de glycane par modulation d'un paramètre de production, ledit procédé comprenant :
la sélection d'un niveau de référence de ladite caractéristique de glycane ;
la sélection d'une valeur pour un paramètre de production pour produire un niveau modulé de ladite caractéristique de glycane ; et
l'application de la valeur sélectionnée dudit paramètre de production dans un processus de fabrication d'une protéine avec une quantité modulée de ladite caractéristique de glycane, où ledit processus de fabrication de la protéine comprend la culture d'une cellule CHO dans un milieu de culture de cellules ;
dans lequel :
ladite caractéristique de glycane est une fucosylation et ladite quantité modulée de ladite caractéristique de glycane est un taux réduit de fucosylation ; et
ledit paramètre de production est la teneur en glucosamine du milieu de culture de cellules et ladite valeur pour ledit paramètre de production est une concentration de glucosamine de 0,001 à 40 mM.

2. Procédé de la revendication 1, dans lequel la glucosamine est ajoutée au milieu de culture de cellules.

3. Procédé de la revendication 1, dans lequel la glucosamine est fournie de façon semi-discontinue à la cellule.

4. Procédé de la revendication 1, dans lequel une enzyme et/ou une substrat est ajouté au milieu de culture de cellules ou fourni de façon semi-discontinue à la cellule de sorte que la glucosamine soit produite.

5. Procédé de la revendication 4, dans lequel le substrat est choisi parmi : la N-acétylglucosamine, la N-acétylglucosamine 6-phosphate, la N-acétylmannosamine et le fructose.

6. Procédé de l'une quelconque des revendications précédentes, dans lequel la protéine est un anticorps.
